(19) Europäisches Patentamt
European Patent Office
Office européen des brevets

(11) Veröffentlichungsnummer: **0 070 802**
**B1**

(12) # EUROPÄISCHE PATENTSCHRIFT

(45) Veröffentlichungstag der Patentschrift: **28.01.87**

(51) Int. Cl.[4]: **C 07 D 239/46**, C 07 D 239/42, C 07 D 251/16, C 07 D 251/22, C 07 D 251/38, C 07 D 251/26, C 07 D 251/46, A 01 N 47/36

(21) Anmeldenummer: **82810298.8**

(22) Anmeldetag: **12.07.82**

(54) **N-Phenylsulfonyl-N'-pyrimidinyl- und -triazinylharnstoffe.**

(30) Priorität: **16.07.81 CH 4667/81**

(43) Veröffentlichungstag der Anmeldung: **26.01.83 Patentblatt 83/4**

(45) Bekanntmachung des Hinweises auf die Patenterteilung: **28.01.87 Patentblatt 87/5**

(84) Benannte Vertragsstaaten: **AT BE CH DE FR GB IT LI NL**

(56) Entgegenhaltungen:
**EP - A - 0 009 419**
**EP - A - 0 015 683**
**EP - A - 0 023 141**
**EP - A - 0 023 422**
**EP - A - 0 024 215**
**EP - A - 0 030 138**
**EP - A - 0 030 140**
**EP - A - 0 044 807**
**EP - A - 0 044 808**
**EP - A - 0 048 143**

(73) Patentinhaber: **CIBA-GEIGY AG, Klybeckstrasse 141, CH-4002 Basel (CH)**

(72) Erfinder: **Föry, Werner, Dr., Benkenstrasse 65, CH-4054 Basel (CH)**
Erfinder: **Gass, Karl, Blumenrain 4, CH-4465 Magden (CH)**
Erfinder: **Meyer, Willy, Talweg 49, CH-4125 Riehen (CH)**
Erfinder: **Schurter, Rolf, Dr., Holzmattstrasse 45, CH-4102 Binningen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 070 802 B1

**Beschreibung**

Die vorliegende Erfindung betrifft neue, herbizid wirksame und pflanzenwuchsregulierende N-Phenylsulfonyl-N'-pyrimidinyl- und -triazinyl-harnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums. Darüber hinaus betrifft die Erfindung auch als Zwischenprodukte hergestellte neue Phenylsulfonamide und N-Phenylsulfonylcarbamate.

Die erfindungsgemässen N-Phenylsulfonyl-N'-pyrimidinyl- und triazinylharnstoffe entsprechen der allgemeinen Formel I

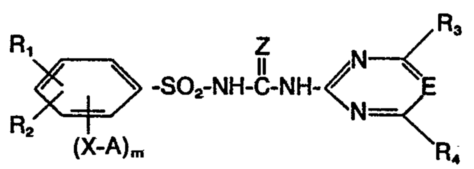 (I)

worin

A einen $C_3$–$C_6$-Alkinylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei,

$R_1$ Wasserstoff, Halogen $C_1$–$C_5$-Alkyl, $C_2$–$C_5$-Alkenyl oder einen Rest $-Y-R_5$,

$R_2$ Wasserstoff, Halogen, $C_1$–$C_5$-Alkyl, $C_2$–$C_5$-Alkenyl, $C_1$–$C_4$-Halogenalkyl, oder einen Rest $-Y-R_5$, $-COOR_6$, $-NO_2$ oder $-CO-NR_7-R_8$,

$R_3$ und $R_4$ unabhängig voneinander Wasserstoff, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, Halogen oder Alkoxyalkyl mit höchstens 4 Kohlenstoffatomen,

$R_5$ und $R_6$ je $C_1$–$C_5$-Alkyl, $C_2$–$C_5$-Alkenyl oder $C_2$–$C_6$-Alkinyl,

$R_7$ und $R_8$ unabhängig voneinander Wasserstoff, $C_1$–$C_5$-Alkyl, $C_2$–$C_5$-Alkenyl oder $C_2$–$C_6$-Alkinyl und

Y Sauerstoff, Schwefel, eine Sulfinyl- oder Sulfonylbrücke bedeuten, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 1514, 1515, 9419, 23 141, 23 422, 24 215, 30 138, 30 140, 44 807, 44 808, 48 143, dem US-Patent Nr. 4 127 405, in der DE-OS 2 715 786 oder in der FR-PS 1 468 747 beschrieben.

In den Definitionen ist unter Alkyl geradkettiges oder verzweigtes Alkyl zu verstehen; z. B.: Methyl, Äthyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Äthoxy, n-Propyloxy, i-Propyloxy und die vier isomeren Butyloxyreste, insbesondere aber Methoxy, Äthoxy oder i-Propoxy.

Beispiele für Alkylthio sind Methylthio, Äthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiel für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel sind die Verbindungen bevorzugt, in denen

a) Z Sauerstoff bedeutet und

b) die Reste $R_3$ und $R_4$ zusammen nicht mehr als 4 Kohlenstoffatome enthalten.

Die Gruppe a) kann aufgeteilt werden in zwei weitere Untergruppen, die aus Verbindungen bestehen, in denen

aa) m die Zahl eins und

ab) m die Zahl zwei bedeutet.

Aus der Gruppe aa) sind die Verbindungen bevorzugt, in denen der Rest –X–A in der 2- oder 3-Position zum Sulfonylrest steht. Hierunter geniessen eine weitere Bevorzugung die Verbindungen, in denen der Rest –X–A in der 2-Position steht.

Aus der Gruppe ab) bilden die Verbindungen eine bevorzugte Gruppe, in denen die beiden Reste –X–A in 2- und 5-Stellung zur Sulfonylgruppe stehen.

Eine weitere Bevorzugung von Verbindungen der oben aufgelisteten Untergruppen der Gruppen aa) und ab) besteht darin, dass die Reste $R_3$ und $R_4$ zusammen höchstens 4 Kohlenstoffatome enthalten. Es bestehen somit die besonders bevorzugten Gruppen von Verbindungen darin, dass in den Verbindungen der Formel I

aab) nur ein Rest –X–A in der 2-Stellung zum Sulfonylrest vorhanden ist, Z Sauerstoff bedeutet und die Reste $R_3$ und $R_4$ zusammen nicht mehr als 4 Kohlenstoffatome enthalten, und

abb) zwei Reste –X–A in 2- und 5-Stellung zum Sulfonylrest vorhanden sind, Z Sauerstoff bedeutet und die Reste $R_3$ und $R_4$ zusammen nicht mehr als 4 Kohlenstoffatome enthalten.

Aus der Gruppe aab) sind die Verbindungen bevorzugt, in denen $R_1$ Wasserstoff bedeutet, und der Rest $R_2$ in der 5- oder 6-Stellung zur Sulfonylgruppe steht.

Von diesen Verbindungen sind wiederum die bevorzugt, in denen $R_2$ für Wasserstoff, Halogen, $C_1$–$C_4$-Alkoxy, Nitro oder $COOR_6$ steht.

Eine weitere Bevorzugung innerhalb der letztgenannten Gruppe geniessen die Verbindungen, in denen $R_2$ Wasserstoff, Fluor, Nitro oder $C_1$–$C_4$-Alkoxy und $R_3$ und $R_4$ jeweils Wasserstoff, $C_1$–$C_4$-Alkyl, $C_1$–$C_4$-Alkoxy, $C_1$–$C_4$-Alkylthio, $C_1$–$C_3$-Halogenalkoxy, Halogen oder Alkoxyalkyl bedeuten, wobei $R_3$ und $R_4$ zusammen höchstens 4 Kohlenstoffatome enthalten.

Unter diesen Verbindungen sind wiederum jene bevorzugt, in denen $R_2$ Wasserstoff und $R_3$ und $R_4$ jeweils $C_1$–$C_4$-Alkyl, $C_1$–$C_4$-Alkoxy, Methylthio, 2,2,2-Trifluoräthoxy, Difluormethoxy, 1,1,2,2-Tetrafluoräthoxy, Halogen oder Alkoxyalkyl bedeuten.

Eine weitere Bevorzugung innerhalb der letztgenannten Gruppe geniessen die Verbindungen, in denen X für Sauerstoff oder Schwefel steht.

Ganz besonders bevorzugt sind hiervon wiederum jene, in denen X für Sauerstoff steht. Innerhalb dieser Gruppe sind die Verbindungen bevorzugt, in denen A für Propargyl steht.

Von den Verbindungen der Formel I, in denen Z für Schwefel steht, sind diejenigen bevorzugt, in denen X Sauerstoff oder Schwefel, $R_3$ und $R_4$ jeweils unabhängig voneinander $C_1$–$C_3$-Alkyl, $C_1$–$C_3$-Alkoxy, $C_1$–$C_3$-Alkylthio, Difluormethoxy oder 2,2,2-Trifluoräthoxy mit zusammen höchstens 4 Kohlenstoffatomen bedeuten und A für $-CH_2-C\equiv CH$, $-CH_3-C\equiv C-CH_3$ oder $-CH_2-CH_2-C\equiv CH$ steht und der Rest –X–A die 2-Stellung besetzt und m die Zahl 1 bedeutet.

Als bevorzugte Einzelverbindungen sind zu nennen:

N-(2-Propargyloxyphenyl-sulfonyl)-N'-(4-methoxy-6-methyl-1,3,5-triazin-2-yl)-harnstoff,

N-(2-Propargyloxyphenyl-sulfonyl)-N'-(4-äthyl-6-methoxy-1,3,5-triazin-2-yl)-harnstoff,

N-(2-Propargyloxyphenyl-sulfonyl)-N'-(4,5-dimethoxy-1,3,5-triazin-2-yl)-harnstoff und

N-(2-Propargyloxyphenyl-sulfonyl)-N'-(4-äthoxy-6-methyl-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

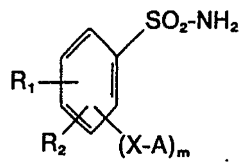

(II),

worin A, $R_1$, $R_2$, X und m die unter Formel I gegebene Bedeutung haben, in Gegenwart einer Base mit einem N-Pyrimidinyl- oder -Triazinylcarbamat der Formel III

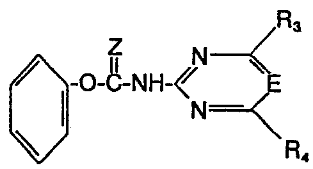

(III)

worin E, $R_3$, $R_4$ und Z die unter Formel I gegebene Bedeutung haben, umsetzt.

Nach einem zweiten Verfahren gelangt man zu Verbindungen der Formel I, indem man ein Phenylsulfonylisocyanat oder -isothiocyanat der Formel IV

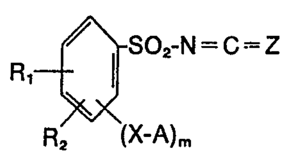

(IV),

worin

A, $R_1$, $R_2$, m, X und Z die unter Formel I gegebene Bedeutung haben, gegebenenfalls in Gegenwart einer Base, mit einem Amin der Formel V

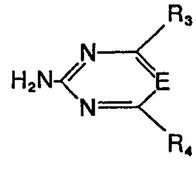

(V),

worin E, $R_3$ und $R_4$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

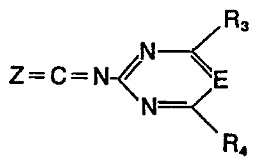

(VI),

worin

E, $R_3$, $R_4$ und Z die unter Formel I gegebene Bedeutung haben, umsetzt.

Schliesslich kann man die Verbindungen der Formel I auch erhalten, indem man ein N-Phenylsulfonylcarbamat der Formel VII

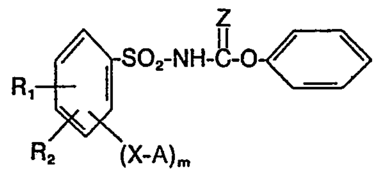

(VII)

worin A, $R_1$, $R_2$, m, X und Z die unter Formel I gegebene Bedeutung haben, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsstoffe der Formel II, IV und VII sind neu und können nach folgenden Methoden hergestellt werden:

Die neuen und als Zwischenprodukte verwendeten Sulfonamide der Formel II werden aus den entsprechenden Anilinen durch Diazotierung und Austausch der Diazogruppe mit Schwefeldioxid in Gegenwart eines Katalysators wie Kupfer -I-chlorid in Salzsäure oder Essigsäure und Umsetzen des entstandenen Phenylsulfonylchlorids mit Ammoniak erhalten.

Die Verbindungen der Formel II können auch durch Sauerstoff- oder Schwefel–Alkinylierung von Hydroxy- oder Thiophenylsulfonamiden mit den entsprechenden Halogeniden resp. Schwefelsäureestern oder durch Umsatz von ortho-Halogenphenylsulfonamiden mit Metall-Alkoholaten resp. Mercaptiden und gegebenenfalls durch deren Oxidation z.B. mit Perjodaten resp. Persäuren zu den entsprechenden Sulfoxiden und Sulfonen erhalten werden.

Ortho-substituierte Hydroxyphenyl- resp. substituierte ortho-Hydroxyphenylsulfonamide der Formel VIII,

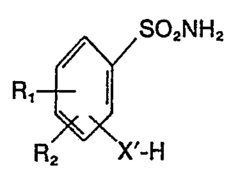

(VIII)

worin $R_1$ und $R_2$ die unter Formel I angegebene Bedeutung haben und X′ für Sauerstoff oder Schwefel steht, als Ausgangsprodukte bestimmter Sulfonamid-Vertreter der Formel II sind aus der Europäischen Patentanmeldung Nr. 44 807 bekannt. Sie können durch Ätherspaltung der entsprechenden $C_1$–$C_4$-Alkoxyphenylsulfonamiden z.B. mit Bortrihalogeniden (solche Umsetzungen sind in der US-Patentschrift 3 904 680 und in J. Am. Chem. Soc. 64, 1128 (1942) beschrieben) oder durch Hydrogenolyse der entsprechenden Benzyloxyphenylsulfonamiden erhalten werden, wie in J. Chem. Soc. 1958, 2903 beschrieben ist.

Die Alkoxyphenylsulfonamide wiederum können aus den entsprechenden Alkoxyaniliden wie bereits erwähnt oder durch Chlorsulfonylierung von Alkoxybenzolen durch Umsatz der erhaltenen Phenylsulfonylchloriden mit Ammoniak gewonnen werden. Solche Reaktionen sind aus J. Am. Chem. Soc. 62, 603 (1940) bekannt geworden.

Die als Zwischenprodukte verwendeten Verbindungen der Formeln II und VII sind neu und speziell für die Synthese von Verbindungen der Formel I entwickelt worden. Diese Zwischenprodukte bilden einen weiteren Aspekt der vorliegenden Erfindung.

Die Phenylsulfonylisocyanate der Formel IV können durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Ähnliche Darstellung sind in «Neuere Methoden der präparativen organischen Chemie», Band VI, 211–229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessender Umsetzung des Dikaliumsalzes mit Phosgen erhalten. Solche Verfahren sind in Arch. Pharm. 199, 174 (1966) beschrieben.

Die N-Phenylsulfonylcarbamate der Formel VII werden durch Umsetzung der Sulfonamide der Formel II mit Diphenylcarbonat in Gegenwart einer Base erhalten. Ähnliche Verfahren sind in der japanischen Patentschrift 61 169 erwähnt.

Die Ausgangsmaterialien der Formeln III, V und VI sind zum Teil bekannt. Neue Verbindungen der Formeln III und VI können nach bekannten Methoden aus entsprechenden Verbindungen der Formel V erhalten werden.

Neue Fluoralkoxy-aminopyrimidine und -triazine der Formel V und ihre Herstellung sowie die Herstellung entsprechender Verbindungen der Formeln III und VI daraus werden in der Europäischen Patentanmeldung Nr. 70 804 beschrieben.

Durch Umsetzung von Aminen der Formel V mit Oxalylchlorid in chlorierten Kohlenwasserstoffen als Lösungsmittel, lassen sich Isocyanate der Formel VI herstellen. Amine der Formel V sind bekannt und zum Teil im Handel erhältlich oder sie können nach bekannten Methoden hergestellt werden, siehe «The Chemistry of Heterocyclic Compounds» Band XIV, Interescience Publishers, New York, London.

Diese Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen $-20^{\circ}$ und $+120\,^{\circ}C$. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Die bevorzugte Kultur ist Getreide wie Weizen, Gerste und Roggen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perenierende Unkräuter bis in die Wurzeln zu schädigen. Die neuen Verbindungen der Formel I wirken bereits bei – im Vergleich zu anderen Herbiziden und Wuchsregulatoren – sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragsteigerung mit Wachstumshemmern beruht darauf, dass die Nährstoffe in stärkerem Masse der Blüten- und Fruchtbildung zugute kommen, während das vegetative Wachstum eingeschränkt wird.

So können z.B. die in der Landwirtschaft in tropischen Gegenden häufig als «cover crops» (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die «cover crops» jedoch nicht zur Konkurrenz für die Kultur werden können.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt

werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z.B. die Na- oder K-Salze oder Öl- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenid, Methylsulfate oder Äthylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979. Sisley and Wood, «Encyclopedia of Surface Active Agents», Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Emulgierbare Konzentrate
Aktiver Wirkstoff:
1 bis 20%, bevorzugt 5 bis 10%
oberflächenaktives Mittel:
5 bis 30%, vorzugsweise 10 bis 20%
flüssiges Trägermittel:
50 bis 94%, vorzugsweise 70 bis 85%.

Stäube
Aktiver Wirkstoff:
0,1 bis 10%, vorzugsweise 0,1 bis 1%
festes Trägermittel:
99,9 bis 90%, vorzugsweise 99,9 bis 99%.

Suspension-Konzentrate
Aktiver Wirkstoff:
5 bis 75%, vorzugsweise 10 bis 50%
Wasser:
94 bis 25%, vorzugsweise 90 bis 30%
oberflächenaktives Mittel:
1 bis 40%, vorzugsweise 2 bis 30%.

Benetzbare Pulver
Aktiver Wirkstoff:
0,5 bis 90%, vorzugsweise 1 bis 80%
oberflächenaktives Mittel:
0,5 bis 20%, vorzugsweise 1 bis 15%
festes Trägermittel:
5 bis 95%, vorzugsweise 15 bis 90%.

Granulate
Aktiver Wirkstoff:
0,5 bis 30%, vorzugsweise 3 bis 15%
festes Trägermittel:
99,5 bis 70%, vorzugsweise 97 bis 85%.

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:
Beispiel 1:

a) 2-Propargyloxyphenylsulfonamid:

Ein Gemisch von 35 g 2-Hydroxyphenyl-sulfonamid, 55,2 g Kaliumcarbonat, 15,5 ml Propargylbromid und 1000 ml Äthylmethylketon wird für 1,5 Stunden zum Rückfluss erhitzt, dann abgekühlt, filtriert und eingedampft. Durch Umkristallisieren des Rückstandes aus Äthylacetat erhält man 30 g 2-Propargyloxyphenylsulfonamid, Smp. 143–145°.

b) N-(2-Propargyloxyphenylsulfonyl)-phenylcarbamat:

Zur Suspension von 3,09 g 55%igem Natriumhydrid in 30 ml absolutem Dimethylformamid lässt man bei einer Temperatur von max. 10° unter einer Stickstoffatmosphäre innerhalb von 10 Minuten eine Lösung von 15,1 g 2-Propargyloxyphenylsulfonamid in 100 ml Dimethylformamid zutropfen. Nach Rühren für 15 Minuten bei Raumtemperatur lässt man innerhalb von 20 Minuten eine Lösung von 15,94 g Diphenylcarbonat in 100 ml Dimethylformamid zur Reaktionsmischung zutropfen und rührt das Gemisch anschliessend für weitere 45 Minuten.

Anschliessend wird das Reaktionsgemisch in einer Mischung von 600 g Eis, 90,5 ml 2N Salzsäure und 600 ml Äthylacetat aufgenommen. Die organische Phase wird zweimal mit Eiswasser gewaschen, über Natriumsulfat getrocknet und eingedampft. Durch Versetzen des öligen Rückstandes mit Äther/Petroläther (1:1) erhält man 21,4 g kristallines N-(2-Propargyloxyphenylsulfonyl)-phenyl-carbamat, Smp. 155–157°.

c) N-(2-Propargyloxyphenylsulfonyl)-N'-(4-Methoxy-6-methyl-1,3,5-triazin-2-yl)-harnstoff:

Eine Mischung von 3,33 g N-(2-Propargyloxyphenylsulfonyl)-phenylcarbamat und 1,4 g 2-Amino-4-methoxy-6-methyl-1,3,5-triazin in 30 ml absolutem Dioxan wird für 45 Minuten zum Rückfluss erhitzt, danach auf Raumtemperatur abgekühlt, filtriert und eingedampft. Durch Verreiben des Rückstandes mit 40 ml Äther erhält man 2,38 g kristallinen
N-(2-Propargyloxyphenylsulfonyl)-N'-(4-methoxy-6-methyl-1,3,5-triazin-2-yl)-harnstoff,
Smp. 157–158°.

Beispiel 2:

a) 5-Methyl-2-propargyloxyphenylsulfonamid:

Ein Gemisch von 24,3 g 2-Hydroxy-5-methylphenylsulfonamid, 36,0 g Kaliumcarbonat, 16,7 g Propargylbromid und 500 ml Acetonitril wird für 4,5 Stunden bei 50 °C gerührt. Das Reaktionsgemisch wird filtriert und eingedampft. Durch Umkristallisieren des Rückstandes aus Äthylacetat erhält man 23,9 g 5-Methyl-2-propargyloxyphenylsulfonamid, Smp. 169–170°.

b) 5-Methyl-2-propargyloxyphenylsulfonylisocyanat:

Eine Mischung von 22,4 g 5-Methyl-2-propargyloxyphenylsulfonamid, 9,9 g n-Butylisocyanat, 0,3 g 1,4-Diazabicyclo(2,2,2)octan und 350 ml absolutem Xylol wird für 30 Minuten zum Rückfluss erhitzt. Anschliessend leitet man für 2 Stunden bei 115–120 °C ca. 20 g Phosgen ein. Nachdem der Phosgenüberschuss durch Einleiten von Stickstoff aus dem Reaktionsgemisch entfernt worden ist, wird dieses auf Raumtemperatur abgekühlt, filtriert und im Vakuum eingedampft. Man erhält so als Rohprodukt 28,1 g 5-Methyl-2-propargyloxyphenylsulfonylisocyanat als braunes Öl. Dieses Rohprodukt wird ohne weitere Reinigung in der Folgestufe eingesetzt.

c) N-(5-Methyl-2-propargyloxyphenylsulfonyl)-N'-(4-chlor-6-methoxypyrimidin-2-yl)-harnstoff:

Zu einer Lösung von 3,7 g 2-Amino-4-chlor-6-methoxy-pyrimidin und 0,1 g 1,4-Diazadicyclo-[2.2.2]octan in 60 ml absolutem Tetrahydrofuran lässt man innerhalb von 10 Minuten bei Raumtemperatur eine Lösung von 9,4 g rohem 5-Methyl-2-propargyloxyphenylsulfonylisocyanat in 50 ml absolutem Tetrahydrofuran zu tropfen. Während dieser Zeit steigt die Temperatur leicht an. Die Reaktionslösung wird für 18 Stunden bei Raumtemperatur gerührt, filtriert und eingedampft. Durch Kristallisieren des Rückstandes aus Äthylacetat erhält man 5,8 g
N-(5-Methyl-2-propargyloxyphenylsulfonyl)-N'-(4-chlor-6-methoxy-pyrimidin-2-yl)-harnstoff,
Smp. 202–204 °C.

In analoger Weise erhält man die in den anschliessenden Tabellen aufgelisteten Zwischen- und Endprodukte.

Tabelle 1

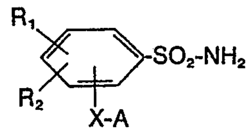

| $R_1$ | $R_2$ | X | A | Stellung von X–A | phys. Daten |
|---|---|---|---|---|---|
| H | H | O | $-CH_2-C\equiv CH$ | 2 | Smp: 143–145° |
| H | H | S | $-CH_2-C\equiv CH$ | 2 | |
| H | H | O | $-CH_2-CH_2-C\equiv CH$ | 2 | |
| H | H | O | $-CH_2-C\equiv C-CH_3$ | 2 | |
| 6–Cl | H | O | $-CH_2-C\equiv CH$ | 2 | |
| 6–Cl | H | O | $-CH_2-C\equiv C-CH_3$ | 2 | |
| 6–Cl | H | O | $-CH_2-CH_2-C\equiv CH$ | 2 | |
| 6–$OCH_3$ | H | O | $-CH_2-C\equiv CH$ | 2 | |
| 6–$OCH_3$ | H | O | $-CH_2-C\equiv C-CH_3$ | 2 | |
| 6–$CH_3$ | H | O | $-CH_2-C\equiv CH$ | 2 | |
| 6–$CH_3$ | H | O | $-CH_2-CH_2-C\equiv CH$ | 2 | |
| 6–$NO_2$ | H | O | $-CH_2-C\equiv CH$ | 2 | |
| 6–$NO_2$ | H | O | $-CH_2-C\equiv C-CH_3$ | 2 | |
| 6–$COOCH_3$ | H | O | $-CH_2-C\equiv CH$ | 2 | |
| 5–Cl | H | O | $-CH_2-C\equiv CH$ | 2 | Smp: 145° |
| 5–Cl | H | O | $-CH_2-CH_2-C\equiv CH$ | 2 | |
| 5–Cl | H | O | $-CH_2-C\equiv C-CH_3$ | 2 | |
| 5–Br | H | O | $-CH_2-C\equiv CH$ | 2 | |
| 5–Br | H | O | $-CH_2-C\equiv C-CH_3$ | 2 | |
| 5–Br | H | O | $-CH_2-CH_2-C\equiv CH$ | 2 | |
| 5–F | H | O | $-CH_2-C\equiv CH$ | 2 | Smp: 156–157° |
| 5–F | H | O | $-CH_2-CH_2-C\equiv CH$ | 2 | |
| 5–F | H | O | $-CH_2-C\equiv C-CH_3$ | 2 | |
| 5–$CH(CH_3)_2$ | H | O | $-CH_2-C\equiv CH$ | 2 | |
| 5–$CH(CH_3)_2$ | H | O | $-CH_2-CH_2-C\equiv CH$ | 2 | |
| 5–$CH(CH_3)_2$ | H | O | $-CH_2-C\equiv C-CH_3$ | 2 | |
| 5–$CH_3$ | H | O | $-CH_2-C\equiv CH$ | 2 | Smp: 169–171° |
| 5–$CH_3$ | H | O | $-CH_2-C\equiv C-CH_3$ | 2 | |
| 5–$CH_3$ | H | O | $-CH_2-CH_2-C\equiv CH$ | 2 | |
| 5–$NO_2$ | H | O | $-CH_2-C\equiv CH$ | 2 | |
| 5–$NO_2$ | H | O | $-CH_2-CH_2-C\equiv CH$ | 2 | |
| 5–$NO_2$ | H | O | $-CH_2-C\equiv C-CH_3$ | 2 | |
| 5–$OCH_3$ | H | O | $-CH_2-C\equiv C-CH_3$ | 2 | |
| 5–$OCH_3$ | H | O | $-CH_2-C\equiv CH$ | 2 | |
| 5–$OCH_3$ | H | O | $-CH_2-CH_2-C\equiv CH$ | 2 | |
| 5–$CON(CH_3)_2$ | H | O | $-CH_2-C\equiv CH$ | 2 | |
| 5–$CON(CH_3)_2$ | H | O | $-CH_2-C\equiv C-CH_3$ | 2 | |
| 5–$COOCH_3$ | H | O | $-CH_2-C\equiv CH$ | 2 | |
| 5–$COOCH_3$ | H | O | $-CH_2-C\equiv C-CH_3$ | 2 | |
| 5–$COOH_3$ | H | O | $-CH_2-CH_2-C\equiv CH$ | 2 | |
| 5–$NO_2$ | 3–$CF_3$ | O | $-CH_2-C\equiv CH$ | 2 | |
| 5–$NO_2$ | 3–Cl | O | $-CH_2-C\equiv CH$ | 2 | |
| 5–$NO_2$ | 3–Cl | O | $-CH_2-C\equiv C-CH_3$ | 2 | |
| 5–$CF_3$ | 3–$NO_2$ | O | $-CH_2-C\equiv CH$ | 2 | |
| 5–$CH_3$ | 3–$CH_3$ | O | $-CH_2-C\equiv CH$ | 2 | |
| 5–$CH_3$ | 3–$CH_3$ | O | $-CH_2-CH_2-C\equiv CH$ | 2 | |
| 5–Cl | 3–$NO_2$ | O | $-CH_2-C\equiv CH$ | 2 | |
| 5–Cl | 3–Cl | O | $-CH_2-C\equiv CH$ | 2 | |
| 5–Cl | 3–Cl | O | $-CH_2-C\equiv C-CH_3$ | 2 | |
| 5–Cl | 3–Cl | O | $-CH_2-CH_2-C\equiv CH$ | 2 | |
| 5–Br | 3–$OCH_3$ | O | $-CH_2-C\equiv CH$ | 2 | |
| 5–Br | 2–$OCH_3$ | O | $-CH_2-C\equiv CH$ | 3 | |
| 3–$OCH_3$ | 5–$COOCH_3$ | O | $-CH_2-C\equiv CH$ | 2 | |
| 2–$OCH_3$ | 5–$COOCH_3$ | O | $-CH_2-C\equiv CH$ | 3 | |

Tabelle 1 (Fortsetzung)

| $R_1$ | $R_2$ | X | A | Stellung von X–A | phys. Daten |
|---|---|---|---|---|---|
| 5–$CH_3$ | 3–Br | O | $-CH_2-C\equiv CH$ | 2 | |
| 5–Br | 3–$NO_2$ | O | $-CH_2-C\equiv CH$ | 2 | |
| 5–Cl | 3–Br | O | $-CH_2-C\equiv CH$ | 2 | |
| 5–Cl | 3–Br | O | $-CH_2-C\equiv C-CH_3$ | 2 | |
| 5–Cl | 3–Br | O | $-CH_2-CH_2-C\equiv CH$ | 2 | |
| 5–$CH(CH_3)_2$ | 3–$CH_3$ | O | $-CH_2-CH_2-C\equiv CH$ | 2 | |
| 5–$CH(CH_3)_2$ | 3–$CH_3$ | O | $-CH_2-C\equiv C-CH_3$ | 2 | |
| 5–$CH(CH_3)_2$ | 3–$CH_3$ | O | $-CH_2-C\equiv CH$ | 2 | |
| 3–$NO_2$ | H | O | $-CH_2-C\equiv CH$ | 2 | |
| 3–$CH_3$ | H | O | $-CH_2-C\equiv CH$ | 2 | |
| 3–$NO_2$ | H | O | $-CH_2-C\equiv CH$ | 2 | |
| 3–Cl | H | O | $-CH_2-C\equiv CH$ | 2 | |
| 3–Cl | H | O | $-CH_2-C\equiv C-CH_3$ | 2 | |
| 3–Cl | H | O | $-CH_2-CH_2-C\equiv CH$ | 2 | |
| 3–$OCH_3$ | H | O | $-CH_2-C\equiv CH$ | 2 | |
| 3–$OCH_3$ | H | O | $-CH_2-C\equiv C-CH_3$ | 2 | |
| 2–$OCH_3$ | H | O | $-CH_2-C\equiv CH$ | 2 | |
| H | H | SO | $-CH_2-C\equiv CH$ | 2 | |
| H | H | $SO_2$ | $-CH_2-C\equiv CH$ | 2 | |
| H | H | SO | $-CH_2-C\equiv C-CH_3$ | 2 | |
| H | H | $SO_2$ | $-CH_2-C\equiv C-CH_2$ | 2 | |
| 2–Cl | H | O | $-CH_2-C\equiv CH$ | 5 | Smp: 149° |
| 6–Cl | 4–Br | O | $-CH_2-C\equiv CH$ | 3 | Smp: 174° |

Tabelle 2

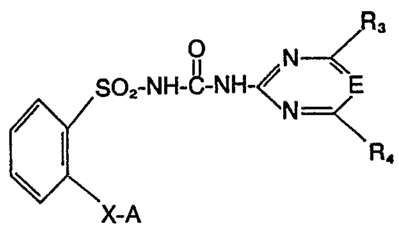

| Nr. | A | $R_3$ | $R_4$ | X | E | phys. Daten |
|---|---|---|---|---|---|---|
| 1 | $-CH_2-C\equiv CH$ | $CH_3$ | $C_2H_5$ | S | N | |
| 2 | $-CH_2-C\equiv CH$ | $OCH_3$ | $C_2H_5$ | S | N | |
| 3 | $-CH_2-C\equiv CH$ | $CH_3$ | $OCH_3$ | S | CH | |
| 4 | $-CH_2-C\equiv CH$ | $OCH_3$ | $OCH_3$ | S | CH | |
| 5 | $-CH_2-C\equiv CH$ | $CH_3$ | $OCH_3$ | SO | N | |
| 6 | $-CH_2-C\equiv CH$ | $OCH_3$ | $OCH_3$ | SO | N | |
| 7 | $-CH_2-C\equiv CH$ | $OCH_3$ | $C_2H_5$ | SO | N | |
| 8 | $-CH_2-C\equiv CH$ | $-CH(CH_3)_2$ | $OCH_3$ | O | CH | Smp: 129–130° |
| 9 | $-CH_2-C\equiv CH$ | $OCH_3$ | $SC_2H_5$ | O | CH | |
| 10 | $-CH_2-C\equiv CH$ | $OCH_3$ | $SC_2H_5$ | O | N | |
| 11 | $-CH_2-C\equiv CH$ | $OCH_3$ | $-SCH(CH_3)_2$ | O | N | |
| 12 | $-CH_2-C\equiv CH$ | CCl | $CH_3$ | O | N | Smp. 179–180° |
| 13 | $-CH_2-C\equiv CH$ | $CH_3$ | Br | O | N | |
| 14 | $-CH_2-C\equiv CH$ | $CHF_2$ | $OCH_3$ | O | N | Smp: 166–167° |
| 15 | $-CH_2-C\equiv CH$ | $CHF_2$ | $CH_3$ | O | N | |
| 16 | $-CH_2-C\equiv CH$ | $-OCH_2CF_3$ | Cl | O | N | |
| 17 | $-CH_2-C\equiv CH$ | $-OCH_2CF_3$ | $OCH_3$ | O | N | Smp: 113–115° |
| 18 | $-CH_2-C\equiv CH$ | $-CH_2CF_3$ | $CH_3$ | O | N | |
| 19 | $-CH_2-C\equiv CH$ | $OC_2H_5$ | $OC_2H_5$ | O | N | Smp: 138–139° |

Tabelle 2 (Fortsetzung)

| Nr. | A | $R_3$ | $R_4$ | X | E | phys. Daten |
|---|---|---|---|---|---|---|
| 20 | $-CH_2-C\equiv CH$ | $OC_2H_5$ | $OC_2H_5$ | O | CH | |
| 21 | $-CH_2-C\equiv CH$ | $C_2H_5$ | $OCH_3$ | O | N | Smp: 134–135° |
| 22 | $-CH_2-C\equiv CH$ | $C_2H_5$ | $OC_2H_5$ | O | N | |
| 23 | $-CH_2-C\equiv CH$ | $CH_3$ | $OC_2H_5$ | O | N | Smp: 129–130° |
| 24 | $-CH_2-C\equiv CH$ | $CH_3$ | $CH_3$ | O | CH | |
| 25 | $-CH_2-C\equiv CH$ | $CH_3$ | $OCH_3$ | O | CH | Smp: 156–159° |
| 26 | $-CH_2-C\equiv CH$ | $CH_3$ | $-OCH_2-CF_3$ | O | N | |
| 27 | $-CH_2-C\equiv CH$ | $CH_3$ | H | O | N | Smp: 186–187° |
| 28 | $-CH_2-C\equiv CH$ | $C_2H_5$ | Cl | O | CH | |
| 29 | $-CH_2-C\equiv CH$ | $CH_3$ | Cl | O | CH | Smp: 225–227° |
| 30 | $-CH_2-C\equiv CH$ | $CH_3$ | $SCH_3$ | O | CH | |
| 31 | $-CH_2-C\equiv CH$ | $CH_3$ | F | O | CH | |
| 32 | $-CH_2-C\equiv CH$ | $CH_3$ | Br | O | CH | |
| 33 | $-CH_2-C\equiv CH$ | $C_2H_5$ | $OC_2H_5$ | O | CH | |
| 34 | $-CH_2-C\equiv CH$ | $C_2H_5$ | $SCH_3$ | O | CH | |
| 35 | $-CH_2-C\equiv CH$ | $CF_3$ | $CH_3$ | O | CH | |
| 36 | $-CH_2-C\equiv CH$ | $CH_2Cl$ | $CH_3$ | O | CH | |
| 37 | $-CH_2-C\equiv CH$ | $CH_2Cl$ | $OCH_3$ | O | CH | |
| 38 | $-CH_2-C\equiv CH$ | $OCH_3$ | Cl | O | CH | Smp: 209–210° |
| 39 | $-CH_2-C\equiv CH$ | Cl | Cl | O | CH | |
| 40 | $-CH_2-C\equiv CH$ | $OCH_3$ | $SCH_3$ | O | CH | |
| 41 | $-CH_2-C\equiv CH$ | $OCH_3$ | $-OCH(CH_3)_2$ | O | CH | |
| 42 | $-CH_2-C\equiv CH$ | $CH_2F$ | $OCH_3$ | O | CH | |
| 43 | $-CH_2-C\equiv CH$ | $CHF_2$ | $CH_3$ | O | CH | Smp: 189–192° |
| 44 | $-CH_2-C\equiv CH$ | $CF_3$ | $OCH_3$ | O | CH | |
| 45 | $-CH_2-C\equiv CH$ | $C_2H_5$ | $-OCH(CH_3)_2$ | O | N | |
| 46 | $-CH_2-C\equiv CH$ | $C_2H_5$ | Cl | O | N | |
| 47 | $-CH_2-C\equiv CH$ | $C_2H_5$ | $SCH_3$ | O | N | |
| 48 | $-CH_2-C\equiv CH$ | $C_2H_5$ | $CH_3$ | O | N | Smp: 132–134° |
| 49 | $-CH_2-C\equiv CH$ | $C_2H_5$ | $C_2H_5$ | O | N | |
| 50 | $-CH_2-C\equiv CH$ | $OCH_3$ | $-OCH(CH_3)_2$ | O | N | Smp: 93–95° |
| 51 | $-CH_2-C\equiv CH$ | $OCH_3$ | $-OCH(CH_3)-CH_2-CH_3$ | O | N | |
| 52 | $-CH_2-C\equiv CH$ | $CH_3$ | $-CH(CH_3)_2$ | O | N | |
| 53 | $-CH_2-C\equiv CH$ | $-CH(CH_3)_2$ | Cl | O | N | Smp: 120–121° |
| 54 | $-CH_2-C\equiv CH$ | $-CH(CH_3)_2$ | $OCH_3$ | O | N | |
| 55 | $-CH_2-C\equiv CH$ | $-CH(CH_3)_2$ | $OC_2H_5$ | O | N | |
| 56 | $-CH_2-C\equiv CH$ | $-CH(CH_3)_2$ | $SCH_3$ | O | N | |
| 57 | $-CH_2-C\equiv CH$ | $CH_2Cl$ | $CH_3$ | O | N | |
| 58 | $-CH_2-C\equiv CH$ | $CH_2Cl$ | $OCH_3$ | O | N | |
| 59 | $-CH_2-C\equiv CH$ | $CH_2F$ | $CH_3$ | O | N | |
| 60 | $-CH_2-C\equiv CH$ | $CH_2F$ | $OCH_3$ | O | N | |
| 61 | $-CH_2-C\equiv CH$ | $CH_2F$ | $OC_2H_5$ | O | N | |
| 62 | $-CH_2-C\equiv CH$ | $-CH_2-OCH_3$ | $C_2H_5$ | O | N | |
| 63 | $-CH_2-C\equiv CH$ | $-CH_2-SCH_3$ | $OCH_3$ | O | N | |
| 64 | $-CH_2-C\equiv CH$ | $-CH_2-SCH_3$ | $CH_3$ | O | N | |
| 65 | $-CH_2-C\equiv CH$ | $-CH_2-SCH_3$ | $SCH_3$ | O | N | |
| 66 | $-CH_2-C\equiv CH$ | $-CH_2-SCH_3$ | Cl | O | N | |
| 67 | $-CH_2-C\equiv CH$ | $-CH_2-SCH_3$ | $OC_2H_5$ | O | N | |
| 68 | $-CH_2-C\equiv CH$ | $SCH_3$ | Cl | O | N | Smp: 139–140° |
| 69 | $-CH_2-C\equiv CH$ | $SCH_3$ | $OCH_3$ | O | N | |
| 70 | $-CH_2-C\equiv CH$ | $SCH_3$ | $C_2H_5$ | O | N | |
| 71 | $-CH_2-C\equiv CH$ | $SCH_3$ | $-OCH(CH_3)_2$ | O | N | |
| 72 | $-CH_2-C\equiv CH$ | $-OCH(CH_3)_2$ | Cl | O | N | |
| 73 | $-CH_2-C\equiv CH$ | $CF_3$ | $OCH_3$ | O | N | |
| 74 | $-CH_2-C\equiv CH$ | $CF_3$ | $CH_3$ | O | N | |
| 75 | $-CH_2-C\equiv CH$ | $CF_3$ | $OC_2H_5$ | O | N | |
| 76 | $-CH_2-C\equiv CH$ | CCl | $OCH_3$ | O | N | |
| 77 | $-CH_2-C\equiv CH$ | $CCl_3$ | $SCH_3$ | O | N | |
| 78 | $-CH_2-C\equiv CH$ | $CH_3$ | Cl | O | N | |

Tabelle 2 (Fortsetzung)

| Nr. | A | $R_3$ | $R_4$ | X | E | phys. Daten |
|---|---|---|---|---|---|---|
| 79 | $-CH_2-C\equiv CH$ | $OCH_3$ | Cl | O | N | Smp: 148–154° |
| 80 | $-CH_2-C\equiv CH$ | $OCH_3$ | F | O | N | |
| 81 | $-CH_2-C\equiv CH$ | $OCH_3$ | Br | O | N | |
| 82 | $-CH_2-C\equiv CH$ | $CH_3$ | F | O | N | |
| 83 | $-CH_2-C\equiv CH$ | $OCH_3$ | $OCH_3$ | O | CH | |
| 84 | $-CH_2-C\equiv CH$ | $CH_3$ | $CH_3$ | O | N | Smp: 175–177° |
| 85 | $-CH_2-C\equiv CH$ | $OCH_3$ | $OC_2H_5$ | O | N | |
| 86 | $-CH_2-C\equiv CH$ | $OCH_3$ | $OC_2H_5$ | O | CH | |
| 87 | $-CH_2-C\equiv CH$ | $-CH_2-OCH_3$ | $OCH_3$ | O | CH | Smp: 162–163° |
| 88 | $-CH_2-C\equiv CH$ | $-CH_2-OCH_3$ | $OCH_3$ | O | N | |
| 89 | $-CH_2-C\equiv CH$ | $C_2H_5$ | $OCH_3$ | O | CH | |
| 90 | $-CH_2-C\equiv CH$ | $-CH_2-OCH_3$ | $CH_3$ | O | N | |
| 91 | $-CH_2-C\equiv CH$ | $CH_3$ | $OCH_3$ | S | N | |
| 92 | $-CH_2-C\equiv CH$ | $OCH_3$ | $OCH_3$ | S | N | |
| 93 | $-CH_2-C\equiv CH$ | $CH_3$ | $OCH_3$ | O | N | Smp: 157–158° |
| 94 | $-CH_2-C\equiv CH$ | $OCH_3$ | $OCH_3$ | O | N | Smp: 130–132° |
| 95 | $-CH_2-C\equiv C-CH_3$ | $CH_2Cl$ | $OCH_3$ | O | N | |
| 96 | $-CH_2-C\equiv C-CH_3$ | $CH_2F$ | $OCH_3$ | O | N | |
| 97 | $-CH_2-C\equiv C-CH_3$ | $CH_3$ | $OCH_3$ | O | CH | |
| 98 | $-CH_2-C\equiv C-CH_3$ | $OCH_3$ | $OCH_3$ | O | CH | |
| 99 | $-CH_2-C\equiv C-CH_3$ | $CH_3$ | $CH_3$ | O | CH | |
| 100 | $-CH_2-C\equiv C-CH_3$ | $C_2H_5$ | $OCH_3$ | O | CH | |
| 101 | $-CH_2-C\equiv C-CH_3$ | $OCH_3$ | Cl | O | CH | |
| 102 | $-CH_2-C\equiv C-CH_3$ | $CH_3$ | Cl | O | CH | |
| 103 | $-CH_2-C\equiv C-CH_3$ | $CF_3$ | $OCH_3$ | O | CH | |
| 104 | $-CH_2-C\equiv C-CH_3$ | $-CH_2-OCH_3$ | $OCH_3$ | O | CH | |
| 105 | $-CH_2-C\equiv C-CH_3$ | Br | $OCH_3$ | O | CH | |
| 106 | $-CH_2-C\equiv C-CH_3$ | Br | $CH_3$ | O | CH | |
| 107 | $-CH_2-C\equiv C-CH_3$ | $OCH_3$ | $SCH_3$ | O | CH | |
| 108 | $-CH_2-C\equiv C-CH_3$ | $CH_3$ | $SCH_3$ | O | CH | |
| 109 | $-CH_2-C\equiv C-CH_3$ | $OCH_3$ | $OC_2H_5$ | O | CH | |
| 110 | $-CH_2-C\equiv C-CH_3$ | $CH_3$ | $CH_3$ | O | N | |
| 111 | $-CH_2-C\equiv C-CH_3$ | $CH_3$ | $OCH_3$ | O | N | |
| 112 | $-CH_2-C\equiv C-CH_3$ | $OCH_3$ | $OCH_3$ | O | N | |
| 113 | $-CH_2-C\equiv C-CH_3$ | $OCH_3$ | $OC_2H_5$ | O | N | |
| 114 | $-CH_2-C\equiv C-CH_3$ | $C_2H_5$ | $OCH_3$ | O | N | |
| 115 | $-CH_2-C\equiv C-CH_3$ | $C_2H_5$ | $CH_3$ | O | N | |
| 116 | $-CH_2-C\equiv C-CH_3$ | Cl | $OCH_3$ | O | N | |
| 117 | $-CH_2-C\equiv C-CH_3$ | $-OCH(CH_3)_2$ | $OCH_3$ | O | N | |
| 118 | $-CH_2-C\equiv C-CH_3$ | $-OCH(CH_3)_2$ | $OCH_3$ | O | N | |
| 119 | $-CH_2-C\equiv C-CH_3$ | $-OCH(CH_3)_2$ | $CH_3$ | O | N | |
| 120 | $-CH_2-C\equiv C-CH_3$ | $OCH_3$ | $SCH_3$ | O | N | |
| 121 | $-CH_2-C\equiv C-CH_3$ | $OC_2H_5$ | $OC_2H_5$ | O | N | |
| 122 | $-CH_2-C\equiv C-CH_3$ | $OCH_3$ | $-CH_2OCH_3$ | O | N | |
| 123 | $-CH_2-CH_2-C\equiv CH$ | $CH_3$ | $OCH_3$ | O | N | |
| 124 | $-CH_2-CH_2-C\equiv CH$ | $CH_3$ | $CH_3$ | O | N | |
| 125 | $-CH_2-CH_2-C\equiv CH$ | $C_2H_5$ | $OCH_3$ | O | N | |
| 126 | $-CH_2-CH_2-C\equiv CH$ | $C_2H_5$ | $CH_3$ | O | N | |
| 127 | $-CH_2-CH_2-C\equiv CH$ | $OCH_3$ | $OCH_3$ | O | N | |
| 128 | $-CH_2-CH_2-C\equiv CH$ | $OCH_3$ | $OC_2H_5$ | O | N | |
| 129 | $-CH_2-CH_2-C\equiv CH$ | $OC_2H_5$ | $OC_2H_5$ | O | N | |
| 130 | $-CH_2-CH_2-C\equiv CH$ | $-CH_2-OCH_3$ | $OCH_3$ | O | N | |
| 131 | $-CH_2-CH_2-C\equiv CH$ | $CH_3$ | $OCH_3$ | O | CH | |
| 132 | $-CH_2-CH_2-C\equiv CH$ | $OCH_3$ | $OCH_3$ | O | CH | |
| 133 | $-CH_2-CH_2-C\equiv CH$ | $CH_3$ | $CH_3$ | O | CH | |
| 134 | $-CH_2-CH_2-C\equiv CH$ | $-CH_2-OCH_3$ | $OCH_3$ | O | CH | |
| 135 | $-CH_2-CH_2-C\equiv CH$ | $CH_3$ | Cl | O | CH | |

Tabelle 2 (Fortsetzung)

| Nr. | A | $R_3$ | $R_4$ | X | E | phys. Daten |
|---|---|---|---|---|---|---|
| 136 | $-CH_2-CH_2-C\equiv CH$ | $OCH_3$ | Cl | O | CH | |
| 137 | $-CH_2-CH_2-C\equiv CH$ | $OCH_3$ | $OC_2H_5$ | O | CH | |
| 138 | $-CH_2C\equiv CH$ | $CH_3$ | $-OCF_2-CHFCl$ | O | CH | Smp: 153–155° |
| 139 | $-CH_2-C\equiv CH$ | $CH_3$ | $-OCF_2-CHF_2$ | O | CH | Smp: 171–173° |

Tabelle 3

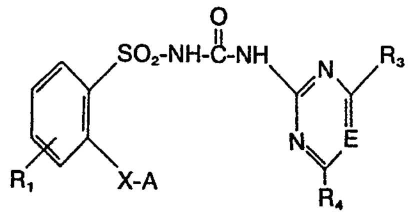

| Nr. | A | $R_1$ | $R_3$ | $R_4$ | X | E | phys. Daten |
|---|---|---|---|---|---|---|---|
| 201 | $-CH_2-C\equiv CH$ | 6–Cl | $CH_3$ | $OCH_3$ | O | N | |
| 202 | $-CH_2-C\equiv CH$ | 6–Cl | $CH_3$ | $OCH_3$ | O | CH | |
| 203 | $-CH_2-C\equiv CH$ | 6–Cl | $C_2H_5$ | $OCH_3$ | O | N | |
| 204 | $-CH_2-C\equiv CH$ | 6–Cl | $OCH_3$ | $OCH_3$ | O | CH | |
| 205 | $-CH_2-C\equiv CH$ | 6–Cl | $OCH_3$ | $OCH_3$ | O | N | |
| 206 | $-CH_2-C\equiv C-CH_3$ | 6–Cl | $OCH_3$ | $CH_3$ | O | N | |
| 207 | $-CH_2-C\equiv C-CH_3$ | 6–Cl | $OCH_3$ | $CH_3$ | O | CN | |
| 208 | $-CH_2-CH_2-C\equiv CH$ | 6–Cl | $OCH_3$ | $CH_3$ | O | N | |
| 209 | $-CH_2-C\equiv CH$ | 6–$OCH_3$ | $CH_3$ | $OCH_3$ | O | CH | |
| 210 | $-CH_2-C\equiv CH$ | 6–$OCH_3$ | $CH_3$ | $OCH_3$ | O | N | |
| 211 | $-CH_2-C\equiv CH$ | 6–$OCH_3$ | $C_2H_5$ | $OCH_3$ | O | N | |
| 212 | $-CH_2-C\equiv CH$ | 6–$OCH_3$ | $OCH_3$ | $OCH_3$ | O | N | |
| 213 | $-CH_2-C\equiv CH$ | 6–$OCH_3$ | $OCH_3$ | $OCH_3$ | O | CH | |
| 214 | $-CH_2-C\equiv CH$ | 6–$CH_3$ | $CH_3$ | $OCH_3$ | O | N | |
| 215 | $-CH_2-C\equiv CH$ | 6–$CH_3$ | $CH_3$ | $CH_3$ | O | CH | |
| 216 | $-CH_2-C\equiv CH$ | 6–$CH_3$ | $OCH_3$ | $OCH_3$ | O | CH | |
| 217 | $-CH_2-C\equiv CH$ | 6–$CH_3$ | $OCH_3$ | $OCH_3$ | O | N | |
| 218 | $-CH_2-C\equiv CH$ | 6–$NO_2$ | $CH_3$ | $OCH_3$ | O | N | |
| 219 | $-CH_2-C\equiv CH$ | 6–$NO_2$ | $CH_3$ | $OCH_3$ | O | CH | |
| 220 | $-CH_2-C\equiv CH$ | 6–$NO_2$ | $OCH_3$ | $OCH_3$ | O | CH | |
| 221 | $-CH_2-C\equiv CH$ | 6–$NO_2$ | $OCH_3$ | $OCH_3$ | O | N | |
| 222 | $-CH_2-C\equiv CH$ | 6–$COOCH_3$ | $OCH_3$ | $CH_3$ | O | CH | |
| 223 | $-CH_2-C\equiv CH$ | 6–$COOCH_3$ | $CH_3$ | $CH_3$ | O | N | |
| 224 | $-CH_2-C\equiv CH$ | 6–$COOCH_3$ | $OCH_3$ | $OCH_3$ | O | N | |
| 225 | $-CH_2-C\equiv CH$ | 6–$COOCH_3$ | $OCH_3$ | $OCH_3$ | O | CH | |
| 226 | $-CH_2-C\equiv CH$ | 5–Cl | $CH_3$ | $OCH_3$ | O | N | Smp. 177° |
| 227 | $-CH_2-C\equiv CH$ | 5–Cl | $CH_3$ | $OCH_3$ | O | CH | Smp. 208° |
| 228 | $-CH_2-C\equiv CH$ | 5–Cl | $OCH_3$ | $OCH_3$ | O | N | |
| 229 | $-CH_2-C\equiv CH$ | 5–Cl | $OCH_3$ | $OCH_3$ | O | N | |
| 230 | $-CH_2-C\equiv CH$ | 5–Cl | $C_2H_5$ | $CH_3$ | O | N | |
| 231 | $-CH_2-CH_2-C\equiv CH$ | 5–Cl | $CH_3$ | $OCH_3$ | O | N | |
| 232 | $-CH_2-CH_2-C\equiv CH$ | 5–Cl | $CH_3$ | $OCH_3$ | O | CH | |
| 233 | $-CH_2-C\equiv C-CH_3$ | 5–Cl | $CH_3$ | $OCH_3$ | O | CH | |
| 234 | $-CH_2-C\equiv C-CH_3$ | 5–Cl | $CH_3$ | $OCH_3$ | O | N | |
| 235 | $-CH_2-C\equiv C-CH_3$ | 5–Cl | $C_2H_5$ | $OCH_3$ | O | N | |
| 236 | $-CH_2-C\equiv C-CH_3$ | 5–Cl | $OCH_3$ | $OCH_3$ | O | N | |
| 237 | $-CH_2-C\equiv C-CH_3$ | 5–Cl | $OCH_3$ | $OCH_3$ | O | CH | |
| 238 | $-CH_2-C\equiv CH$ | 5–F | $CH_3$ | $OCH_3$ | O | N | Smp. 158° |
| 239 | $-CH_2-C\equiv CH$ | 5–F | $CH_3$ | $OCH_3$ | O | CH | Smp. 211° (Zers.) |

Tabelle 3 (Fortsetzung)

| Nr. | A | $R_1$ | $R_3$ | $R_4$ | X | E | phys. Daten |
|---|---|---|---|---|---|---|---|
| 240 | $-CH_2-C\equiv CH$ | 5–F | $OCH_3$ | $C_2H_5$ | O | N | |
| 241 | $-CH_2-C\equiv CH$ | 5–F | $OCH_3$ | $OCH_3$ | O | CH | |
| 242 | $-CH_2-C\equiv CH$ | 5–$NO_2$ | $OCH_3$ | $OCH_3$ | O | N | |
| 243 | $-CH_2-C\equiv CH$ | 5–$NO_2$ | $CH_3$ | $CH_3$ | O | N | |
| 244 | $-CH_2-C\equiv CH$ | 5–$NO_2$ | $CH_3$ | $OCH_3$ | O | CH | |
| 245 | $-CH_2-C\equiv CH$ | 5–$CH(CH_3)_2$ | $CH_3$ | $OCH_3$ | O | N | |
| 246 | $-CH_2-C\equiv CH$ | 5–$CH(CH_3)_2$ | $CH_3$ | $OCH_3$ | O | CH | |
| 247 | $-CH_2-C\equiv CH$ | 5–$CH(CH_3)_2$ | $C_2H_5$ | $OCH_3$ | O | N | |
| 248 | $-CH_2-C\equiv CH$ | 5–$CH(CH_3)_2$ | $OCH_3$ | $OCH_3$ | O | N | |
| 249 | $-CH_2-CH_2-C\equiv CH$ | 5–$CH(CH_3)_2$ | $OCH_3$ | $OCH_3$ | O | N | |
| 250 | $-CH_2-CH_2-C\equiv CH$ | 5–$CH(CH_3)_2$ | $OCH_3$ | $CH_3$ | O | N | |
| 251 | $-CH_2-CH_2-C\equiv CH$ | 5–$CH(CH_3)_2$ | $OCH_3$ | $OCH_3$ | O | CH | |
| 252 | $-CH_2-C\equiv C-CH_3$ | 5–$CH(CH_3)_2$ | $OCH_3$ | $OCH_3$ | O | N | |
| 253 | $-CH_2-C\equiv C-CH_3$ | 5–$CH(CH_3)_2$ | $OCH_3$ | $OCH_3$ | O | CH | |
| 254 | $-CH_2-C\equiv C-CH_3$ | 5–$CH(CH_3)_2$ | $OCH_3$ | $CH_3$ | O | N | |
| 255 | $-CH_2-C\equiv C-CH_3$ | 5–$CH(CH_3)_2$ | $OCH_3$ | $CH_3$ | O | CH | |
| 256 | $-CH_2-C\equiv CH$ | 5–$CH_3$ | $CH_3$ | $OCH_3$ | O | N | |
| 257 | $-CH_2-C\equiv CH$ | 5–$CH_3$ | $CH_3$ | $OCHF_2$ | O | CH | Smp: 201–203° |
| 258 | $-CH_2-C\equiv CH$ | 5–$CH_3$ | $C_2H_5$ | $OCH_3$ | O | N | |
| 259 | $-CH_2-C\equiv CH$ | 5–$CH_3$ | $OCH_3$ | $OCH_3$ | O | N | |
| 260 | $-CH_2-C\equiv CH$ | 5–$CH_3$ | $OCH_3$ | $OCH_3$ | O | CH | |
| 261 | $-CH_2-C\equiv CH$ | 5–$OCH_3$ | $CH_3$ | $OCH_3$ | O | CH | |
| 262 | $-CH_2-C\equiv CH$ | 5–$OCH_3$ | $OCH_3$ | $OCH_3$ | O | CH | |
| 263 | $-CH_2-C\equiv CH$ | 5–$OCH_3$ | $OCH_3$ | $OCH_3$ | O | N | |
| 264 | $-CH_2-C\equiv CH$ | 5–$OCH_3$ | $OCH_3$ | $C_2H_5$ | O | N | |
| 265 | $-CH_2-C\equiv CH$ | 5–$COOCH_3$ | $CH_3$ | $OCH_3$ | O | N | |
| 266 | $-CH_2-C\equiv CH$ | 5–$COOCH_3$ | $CH_3$ | $OCH_3$ | O | CH | |
| 267 | $-CH_2-C\equiv CH$ | 5–$COOCH_3$ | $OCH_3$ | $OCH_3$ | O | N | |
| 268 | $-CH_2-C\equiv CH$ | 5–$COOCH_3$ | $OCH_3$ | $OCH_3$ | O | CH | |
| 269 | $-CH_2-C\equiv CH$ | 3–$NO_2$ | $CH_3$ | $OCH_3$ | O | CH | |
| 270 | $-CH_2-C\equiv CH$ | 3–$NO_2$ | $CH_3$ | $OCH_3$ | O | N | |
| 271 | $-CH_2-C\equiv CH$ | 3–$NO_2$ | $C_2H_5$ | $OCH_3$ | O | N | |
| 272 | $-CH_2-C\equiv CH$ | 3–$NO_2$ | $OCH_3$ | $OCH_3$ | O | N | |
| 273 | $-CH_2-C\equiv CH$ | 3–$OCH_3$ | $CH_3$ | $OCH_3$ | O | N | |
| 274 | $-CH_2-C\equiv CH$ | 3–$OCH_3$ | $CH_3$ | $OCH_3$ | O | CH | |
| 275 | $-CH_2-C\equiv CH$ | 3–$OCH_3$ | $OCH_3$ | $OCH_3$ | O | CH | |
| 276 | $-CH_2-C\equiv CH$ | 3–$OCH_3$ | $C_2H_5$ | $OCH_3$ | O | N | |
| 277 | $-CH_2-C\equiv CH$ | 3–$OCH_3$ | $OCH_3$ | $OCH_3$ | O | N | |
| 278 | $-CH_2-C\equiv CH$ | 3–$CH_3$ | $OCH_3$ | $CH_3$ | O | CH | |
| 279 | $-CH_2-C\equiv CH$ | 3–$CH_3$ | $C_2H_5$ | $CH_3$ | O | N | |
| 280 | $-CH_2-C\equiv CH$ | 3–$CH_3$ | $OCH_3$ | $CH_3$ | O | N | |
| 281 | $-CH_2-C\equiv CH$ | 3–$CH_3$ | $OCH_3$ | $CH_3$ | O | CH | |
| 282 | $-CH_2-C\equiv CH$ | 3–Cl | $OCH_3$ | $CH_3$ | O | CH | |
| 283 | $-CH_2-C\equiv CH$ | 3–Cl | $OCH_3$ | $CH_3$ | O | N | |
| 284 | $-CH_2-C\equiv CH$ | 3–Cl | $OCH_3$ | $OCH_3$ | O | N | |
| 285 | $-CH_2-C\equiv CH$ | 3–Cl | $OCH_3$ | $OCH_3$ | O | CH | |
| 286 | $-CH_2-C\equiv CH$ | 5–Br | $OCH_3$ | $CH_3$ | O | N | Smp. 187–188° |
| 287 | $-CH_2-C\equiv CH$ | 5–$CH_3$ | $CH_3$ | $OCH_3$ | O | N | |
| 288 | $-CH_2-C\equiv CH$ | 5–$CH_3$ | $OCH_3$ | Cl | O | CH | Smp. 202–204° |
| 289 | $-CH_2-C\equiv CH$ | 5–F | $OCH_3$ | $OCH_3$ | O | N | Smp. 182–183° |
| 290 | $-CH_2-C\equiv CH$ | 5–Cl | $OCHF_2$ | $CH_3$ | O | CH | Smp: 175–176° (Zers.) |
| 291 | $-CH_2-C\equiv CH$ | 5–F | $OCHF_2$ | $CH_3$ | O | CH | Smp: 175° |
| 292 | $-CH_2-C\equiv CH$ | H | $OCHF_2$ | $OCH_3$ | O | CH | |
| 293 | $-CH_2-C\equiv CH$ | H | $OCHF_2$ | Cl | O | CH | |
| 294 | $-CH_2-C\equiv CH$ | H | $CH_3$ | $-OCF_2-CHF_2$ | O | CH | |
| 295 | $-CH_2-C\equiv CH$ | H | $OCH_3$ | $-OCF_2-CHF_2$ | O | CH | |
| 296 | $-CH_2-C\equiv CH$ | H | Cl | $-OCF_2-CHF_2$ | O | CH | |
| 297 | $-CH_2-C\equiv CH$ | H | $CH_3$ | $-OCF_2-CHFCl$ | O | CH | |
| 298 | $-CH_2-C\equiv CH$ | H | $OCH_3$ | $-OCF_2-CHFCl$ | O | CH | |

Tabelle 3 (Fortsetzung)

| Nr. | A | $R_1$ | $R_3$ | $R_4$ | X | E | phys. Daten |
|---|---|---|---|---|---|---|---|
| 299 | $-CH_2-C\equiv CH$ | H | Cl | $-OCF_2-CHFCl$ | O | CH | |
| 300 | $-CH_2-C\equiv CH$ | 5–Cl | $CH_3$ | $CH_3$ | O | CH | Smp. 206–208° (Zers.) |
| 301 | $-CH_2-C\equiv CH$ | 5–F | $CH_3$ | $CH_3$ | O | CH | Smp. 219–220° (Zers.) |

Tabelle 4

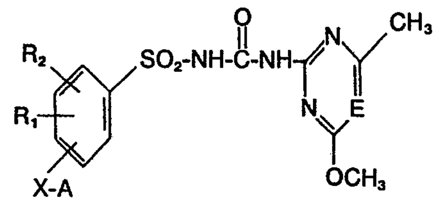

| Nr. | A | Stellung von X–A | $R_1$ | $R_2$ | X | E | phys. Daten |
|---|---|---|---|---|---|---|---|
| 401 | $-CH_2-C\equiv CH$ | 5 | $2-CH_3$ | H | O | N | |
| 402 | $-CH_2-C\equiv C-CH_3$ | 5 | $2-CH_3$ | H | O | CH | |
| 403 | $-CH_2CH_2-C\equiv CH$ | 5 | $2-CH_3$ | H | O | CH | |
| 404 | $-CH_2CH_2-C\equiv CH$ | 5 | $2-CH_3$ | H | O | N | |
| 405 | $-CH_2-C\equiv C-CH_3$ | 5 | $2-CH_3$ | H | O | N | |
| 406 | $-CH_2-C\equiv CH$ | 5 | $2-CH_3$ | H | O | CH | |
| 407 | $-CH_2-C\equiv CH$ | 5 | 2–Cl | H | O | N | Smp: 175–176° |
| 408 | $-CH_2-C\equiv CH$ | 5 | 2–Cl | H | O | CH | |
| 409 | $-CH_2-C\equiv C-CH_3$ | 5 | 2–Cl | H | O | CH | |
| 410 | $-CH_2-C\equiv C-CH_3$ | 5 | 2–Cl | H | O | N | |
| 411 | $-CH_2-CH_2-C\equiv CH$ | 5 | 2–Cl | H | O | N | |
| 412 | $-CH_2-CH_2-C\equiv CH$ | 5 | 2–Cl | H | O | CH | |
| 413 | $-CH_2-CH_2-C\equiv CH$ | 5 | $2-CH_3$ | H | O | N | |
| 414 | $-CH_2-C\equiv CH$ | 3 | $2-CH_3$ | H | O | CH | |
| 415 | $-CH_2-C\equiv C-CH_3$ | 3 | $2-CH_3$ | H | O | CH | |
| 416 | $-CH_2-C\equiv CH$ | 3 | $2-CH_3$ | H | O | N | |
| 417 | $-CH_2-C\equiv CH$ | 3 | $2-OCH_3$ | H | O | CH | |
| 418 | $-CH_2-C\equiv CH$ | 3 | $2-OCH_3$ | H | O | N | |
| 419 | $-CH_2-C\equiv C-CH_3$ | 3 | $2-OCH_3$ | H | O | N | |
| 420 | $-CH_2-C\equiv C-CH_3$ | 3 | $2-OCH_3$ | H | O | CH | |
| 421 | $-CH_2-CH_2-C\equiv CH$ | 3 | $2-OCH_3$ | H | O | CH | |
| 422 | $-CH_2-CH_2-C\equiv CH$ | 3 | $2-OCH_3$ | H | O | N | |
| 423 | $-CH_2-C\equiv CH$ | 5 | $2-OCH_3$ | H | O | N | |
| 424 | $-CH_2-C\equiv CH$ | 5 | $2-OCH_3$ | H | O | CH | |
| 425 | $-CH_2-C\equiv C-CH_3$ | 5 | $2-OCH_3$ | H | O | CH | |
| 426 | $-CH_2-C\equiv C-CH_3$ | 5 | $2-OCH_3$ | H | O | N | |
| 427 | $-CH_2-CH_2-C\equiv CH$ | 5 | $2-OCH_3$ | H | O | N | |
| 428 | $-CH_2-CH_2-C\equiv CH$ | 5 | $2-OCH_3$ | H | O | CH | |
| 429 | $-CH_2-C\equiv CH$ | 3 | 5–Br | $2-OCH_3$ | O | N | |
| 430 | $-CH_2-C\equiv CH$ | 3 | 5–Br | $2-OCH_3$ | O | CH | |
| 431 | $-CH_2-C\equiv C-CH_3$ | 3 | 5–Br | $2-OCH_3$ | O | CH | |
| 432 | $-CH_2-C\equiv C-CH_3$ | 3 | 5–Br | $2-OCH_3$ | O | N | |
| 433 | $-CH_2-C\equiv CH$ | 3 | $5-COOCH_3$ | $2-OCH_3$ | O | N | |
| 434 | $-CH_2-C\equiv CH$ | 3 | $5-COOCH_3$ | $2-OCH_3$ | O | CH | |
| 435 | $-CH_2-CH_2-C\equiv CH$ | 3 | $5-COOCH_3$ | $2-OCH_3$ | O | CH | |
| 436 | $-CH_2-CH_2-C\equiv CH$ | 3 | $5-COOCH_3$ | $2-OCH_3$ | O | N | |
| 437 | $-CH_2-C\equiv CH$ | 3 | $5-COOCH_3$ | $2-OCH_3$ | O | N | |
| 438 | $-CH_2-C\equiv CH$ | 3 | $5-COOCH_3$ | $2-OCH_3$ | O | CH | |

Tabelle 4 (Fortsetzung)

| Nr. | A | Stellung von X–A | $R_1$ | $R_2$ | X | E | phys. Daten |
|---|---|---|---|---|---|---|---|
| 439 | $-CH_2-C\equiv CH$ | 2 | $5-NO_2$ | $3-CF_3$ | O | N | |
| 440 | $-CH_2-C\equiv CH$ | 2 | $5-NO_2$ | $3-CF_3$ | O | CH | |
| 441 | $-CH_2-C\equiv CH$ | 2 | $5-NO_2$ | 3–Cl | O | CH | |
| 442 | $-CH_2-C\equiv CH$ | 2 | $5-NO_2$ | 3–Cl | O | N | |
| 443 | $-CH_2-C\equiv C-CH_3$ | 2 | $5-NO_2$ | 3–Cl | O | N | |
| 444 | $-CH_2-C\equiv C-CH_3$ | 2 | $5-NO_2$ | 3–Cl | O | CH | |
| 445 | $-CH_2-C\equiv CH$ | 2 | $5-CF_3$ | $3-NO_2$ | O | CH | |
| 446 | $-CH_2-C\equiv CH$ | 2 | $5-CF_3$ | $3-NO_2$ | O | N | |
| 447 | $-CH_2-C\equiv CH$ | 2 | $5-CH_3$ | $3-CH_3$ | O | N | |
| 448 | $-CH_2-C\equiv CH$ | 2 | $5-CH_3$ | $3-CH_3$ | O | CH | |
| 449 | $-CH_2-CH_2-C\equiv CH$ | 2 | $5-CH_3$ | $3-CH_3$ | O | CH | |
| 450 | $-CH_2-CH_2-C\equiv CH$ | 2 | $5-CH_3$ | $3-CH_3$ | O | N | |
| 451 | $-CH_2-C\equiv CH$ | 2 | 5–Cl | $3-NO_2$ | O | N | |
| 452 | $-CH_2-C\equiv CH$ | 2 | 5–Cl | $3-NO_2$ | O | CH | |
| 453 | $-CH_2-C\equiv C-CH_3$ | 2 | 5–Cl | $3-NO_2$ | O | CH | |
| 454 | $-CH_2-C\equiv C-CH_3$ | 2 | 5–Cl | $3-NO_2$ | O | N | |
| 455 | $-CH_2-C\equiv CH$ | 2 | 5–Cl | 3–Cl | O | N | |
| 456 | $-CH_2-C\equiv CH$ | 2 | 5–Cl | 3–Cl | O | CH | |
| 457 | $-CH_2-C\equiv C-CH_3$ | 2 | 5–Cl | 3–Cl | O | CH | |
| 458 | $-CH_2-C\equiv C-CH_3$ | 2 | 5–Cl | 3–Cl | O | N | |
| 459 | $-CH_2-CH_2-C\equiv CH$ | 2 | 5–Cl | 3–Cl | O | N | |
| 460 | $-CH_2-CH_2-C\equiv CH$ | 2 | 5–Cl | 3–Cl | O | CH | |
| 461 | $-CH_2-CH_2-C\equiv CH$ | 2 | 5–Br | $3-OCH_3$ | O | N | |
| 462 | $-CH_2-CH_2-C\equiv CH$ | 2 | 5–Br | $3-OCH_3$ | O | CH | |
| 463 | $-CH_2-C\equiv CH$ | 3 | 5–Br | $2-OCH_3$ | O | CH | |
| 464 | $-CH_2-C\equiv CH$ | 3 | 5–Br | $2-OCH_3$ | O | N | |
| 465 | $-CH_2-C\equiv CH$ | 2 | $5-COOCH_3$ | $3-OCH_3$ | O | N | |
| 466 | $-CH_2-C\equiv CH$ | 2 | $5-COOCH_3$ | $3-OCH_3$ | O | CH | |
| 467 | $-CH_2-C\equiv C-CH_3$ | 3 | $5-COOCH_3$ | $2-OCH_3$ | O | CH | |
| 468 | $-CH_2-C\equiv C-CH_3$ | 3 | $5-COOCH_3$ | $2-OCH_3$ | O | N | |
| 469 | $-CH_2-CH_2-C\equiv CH$ | 3 | $5-COOCH_3$ | $2-OCH_3$ | O | N | |
| 470 | $-CH_2-CH_2-C\equiv CH$ | 3 | $5-COOCH_3$ | $2-OCH_3$ | O | CH | |
| 471 | $-CH_2-CH_2-C\equiv CH$ | 3 | $5-CH_3$ | 3–Br | O | CH | |
| 472 | $-CH_2-CH_2-C\equiv CH$ | 2 | $5-CH_3$ | 3–Br | O | N | |
| 473 | $-CH_2-C\equiv CH$ | 2 | $5-CH_3$ | 3–Br | O | N | |
| 474 | $-CH_2-C\equiv CH$ | 2 | $5-CH_3$ | 3–Br | O | CH | |
| 475 | $-CH_2-C\equiv CH$ | 2 | 5–Br | $3-NO_2$ | O | N | |
| 476 | $-CH_2-C\equiv CH$ | 2 | 5–Br | $3-NO_2$ | O | CH | |
| 477 | $-CH_2-C\equiv CH$ | 2 | 5–Cl | 3–Br | O | N | |
| 478 | $-CH_2-C\equiv C-CH_3$ | 2 | 5–Cl | 3–Br | O | N | |
| 479 | $-CH_2-C\equiv C-CH_3$ | 2 | 5–Cl | 3–Br | O | CH | |
| 480 | $-CH_2-C\equiv CH$ | 2 | 5–Cl | 3–Br | O | CH | |
| 481 | $-CH_2-CH_2-C\equiv CH$ | 2 | 5–Cl | 3–Br | O | N | |
| 482 | $-CH_2-C\equiv CH$ | 5 | $2-NO_2$ | H | O | CH | |
| 483 | $-CH_2-C\equiv CH$ | 5 | $2-NO_2$ | H | O | N | |
| 484 | $-CH_2-C\equiv CH$ | 5 | 2–Cl | H | O | N | |
| 485 | $-CH_2-C\equiv CH$ | 5 | 2–Cl | H | O | CH | |
| 486 | $-CH_2-C\equiv C-CH_3$ | 5 | 2–Cl | H | O | CH | |
| 487 | $-CH_2-C\equiv C-CH_3$ | 5 | 2–Cl | H | O | N | |
| 488 | $-CH_2-CH_2-C\equiv CH$ | 5 | 2–Cl | H | O | N | |
| 489 | $-CH_2-CH_2-C\equiv CH$ | 5 | 2–Cl | H | O | CH | |

Formulierungsbeispiele
Beispiel 3:
Formulierungsbeispiele für Wirkstoffe der Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wir mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele
Beispiel 4:
Nachweis der Herbizidwirkung vor dem Auflaufen der Pflanzen

Im Gewächshaus werden Pflanzensamen in Blumentöpfe von 12–15 cm Durchmesser gesät. Unmittelbar danach wird die Erdoberfläche mit einer wässrigen Dispersion oder Lösung der Wirkstoffe behandelt. Es werden Konzentrationen von 4 kg Wirkstoffmengen pro Hektar angewendet. Die Töpfe werden dann im Gewächshaus bei einer Temperatur von 22–25 °C und 50–70% relativer Luftfeuchtigkeit gehalten. Nach 3 Wochen wird der Versuch ausgewertet und die Wirkung auf die Versuchspflanzen nach dem folgenden Massstab bewertet:

1: Pflanze nicht gekeimt oder total abgestorben
2–3: sehr starke Wirkung
4–6: mittlere Wirkung
7–8 schwache Wirkung
9: keine Wirkung (wie unbehandelte Kontrolle).

Pre-emergente Wirkung

Aufwandmenge: 4 kg Wirksubstanz/Hektar

| Verb. Nr. | Avena | Setaria | Sinapis | Stellaria |
|---|---|---|---|---|
| 8 | 6 | 6 | 2 | 2 |
| 19 | 2 | 2 | 2 | 2 |
| 21 | 3 | 3 | 2 | 2 |
| 23 | 2 | 2 | 2 | 2 |
| 29 | 6 | 4 | 3 | 4 |
| 38 | 3 | 3 | 2 | 2 |
| 48 | 6 | 5 | 2 | 2 |
| 68 | 5 | 5 | 2 | 2 |
| 84 | 4 | 4 | 2 | 2 |
| 87 | 4 | 2 | 2 | 2 |
| 93 | 2 | 1 | 2 | 1 |

| | | | | |
|---|---|---|---|---|
| 94 | 2 | 3 | 2 | 2 |
| 226 | 5 | 5 | 2 | 2 |
| 227 | 5 | 6 | 2 | 2 |
| 238* | 4 | 5 | 2 | 2 |
| 239* | 3 | 4 | 2 | 2 |
| 286 | 7 | 8 | 2 | 2 |
| 289* | 6 | 6 | 2 | 2 |
| 407 | 6 | 6 | 3 | 3 |

* Aufwandmenge: 0,5 kg AS/ha

Beispiel 5:
Nachweis der Selektivität bei Vorauflaufanwendung

In der gleichen Versuchsanordnung wie im Beispiel 4 werden eine grössere Anzahl von Pflanzensamen mit verschiedenen Aufwandmengen an Wirksubstanz behandelt. Die Auswertung erfolgte nach dem gleichen Massstab.

pre-emergente Wirkung:

| Wirkung | Verb. Nr. 23 | | Verb. Nr. 93 | |
|---|---|---|---|---|
| Aufwandmenge kg AS/ha Testpflanze | 0,125 | 0,06 | 0,125 | 0,06 |
| Weizen | 9 | 9 | 8 | 9 |
| Alopecurus myos | 2 | 3 | 2 | 2 |
| Echinochloa c.g. | 3 | 3 | 2 | 2 |
| Rottboellia ex. | 8 | 9 | 4 | 4 |
| Abutilon | 2 | 2 | 2 | 2 |
| Xanthium Sp. | 2 | 3 | 6 | 6 |
| Chenopodium Sp. | 3 | 3 | 2 | 2 |
| Ipomoea | 1 | 2 | 3 | 2 |
| Sinapis | 2 | 2 | 2 | 2 |
| Galium aparine | 2 | 2 | 1 | 1 |
| Viola tricolor | 3 | 4 | 2 | 2 |

Beispiel 6:
Nachweis der Herbizidwirkung nach dem Auflaufen der Pflanzen (Kontaktwirkung)

Eine Anzahl Unkräuter und Kulturpflanzen, sowohl monokotyle wie dikotyle, wurden nach dem Auflaufen im 4- bis 6-Blattstadium mit einer wässrigen Wirkstoffdispersion in Dosierungen von 4 kg/AS/ha gespritzt und dann bei 24° bis 26 °C und 45–60% relativer Luftfeuchtigkeit gehalten. 15 Tage nach der Behandlung wird der Versuch ausgewertet und nach dem gleichen Massstab wie im pre-emergenten Versuch bewertet.

post-emergente Wirkung

Aufwandmenge: 4 kg Wirksubstanz/Hektar

| Verb. Nr. | Avena | Setaria | Lolium | Solanum | Sinapis | Stellaria | Phaseolus |
|---|---|---|---|---|---|---|---|
| 8 | 8 | 8 | 3 | 3 | 2 | 3 | 3 |
| 19 | 2 | 4 | 3 | 2 | 2 | 3 | 3 |
| 21 | 5 | 4 | 4 | 2 | 2 | 2 | 4 |
| 23 | 2 | 3 | 2 | 2 | 2 | 2 | 2 |
| 38 | 4 | 5 | 2 | 2 | 2 | 2 | 3 |
| 48 | 6 | 5 | 4 | 2 | 2 | 2 | 4 |
| 68 | 6 | 6 | 2 | 3 | 2 | 2 | 3 |
| 84 | 6 | 5 | 2 | 2 | 2 | 2 | 3 |
| 87 | 5 | 5 | 2 | 3 | 2 | 3 | 4 |
| 93 | 4 | 2 | 4 | 2 | 2 | 3 | 3 |
| 94 | 4 | 3 | 2 | 2 | 2 | 2 | 4 |
| 226 | 7 | 7 | 3 | 3 | 3 | 3 | 4 |
| 227 | 7 | 8 | 4 | 3 | 3 | 4 | 4 |
| 238* | 7 | 6 | 3 | 2 | 2 | 2 | 3 |
| 239* | 4 | 4 | 2 | 3 | 2 | 3 | 3 |
| 289* | 9 | 9 | 3 | 4 | 2 | 2 | 4 |

* Aufwandmenge: 0,5 kg AS/ha

Beispiel 7:
Nachweis der Selektivität bei Nachauflaufanwendung

In der gleichen Versuchsanordnung wie im Beispiel 6 werden eine grössere Anzahl von Pflanzen mit verschiedenen Aufwandmengen an Wirksubstanz behandelt. Die Auswertung erfolgte nach dem in Beispiel 4 angegebenen Massstab.

post-emergente Wirkung

| Wirkung | Verb. Nr. 23 | | Verb. Nr. 38 | | Verb. Nr. 93 | |
|---|---|---|---|---|---|---|
| Aufwandmenge kg AS/ha Testpflanze | 0,125 | 0,06 | 0,125 | 0,06 | 0,125 | 0,06 |
| Weizen | 9 | 9 | 9 | 9 | 8 | 9 |
| Mais | 3 | 3 | 4 | 5 | 9 | 9 |
| Reis trocken | 4 | 6 | 6 | 7 | 9 | 9 |
| Alopecurus myos. | 3 | 3 | 4 | 4 | 1 | 2 |
| Cyperus escul. | 9 | 9 | 3 | 3 | 4 | 9 |
| Soja | 2 | 2 | 6 | 6 | 9 | 9 |
| Baumwolle | 4 | 4 | 7 | 7 | 9 | 9 |
| Sinapis | 2 | 3 | 2 | 2 | 1 | 1 |
| Galium aparine | 4 | 4 | 4 | 4 | 1 | 1 |
| Viola tricolor | 3 | 3 | 4 | 4 | 2 | 2 |

Beispiel 8:
Nachweis der Keimhemmung an Lagerkartoffeln.

Eine Anzahl im Handel erhältliche Kartoffeln der Sorte «Urgenta» ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21 °C im Dunkeln bei 50% relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt. Die erfindungsgemässen Verbindungen zeigten in diesem Versuch eine vollständige Verhinderung der Keimbildung. Gleichzeitig betrug der Gewichtsverlust der Kartoffeln weniger als 10% des Gewichtsverlustes der Kontrollkartoffeln.

Beispiel 9:
Nachweis der Wuchshemmung bei tropischen Bodendeckern-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70% relativer Luftfeuchtigkeit und 6000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20% des Neuzuwachses bei unbehandelten Kontrollpflanzen) ohne dass dabei die Versuchspflanzen geschädigt wurden.

Beispiel 10:
Wuchsregulierung an Sojabohnen

In Kunststoffbehältern mit einem Erde-Torf-Sandgemisch im Verhältnis 6:3:1 werden Sojabohnen der Sorte «Hark» angesät und in eine Klimakammer gegeben. Durch optimale Temperaturwahl, Beleuchtung, Düngerzugabe und Bewässerung entwickeln sich die Pflanzen nach ca. 5 Wochen bis zum 5–6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten am Haupttrieb.

**Patentansprüche für die Vertragsstaaten BE, CH, DE, FR, GB, IT, LI, NL**

1. N-Phenylsulfonyl-N′-pyrimidinyl- und -triazinyl-harnstoffe der allgemeinen Formel I

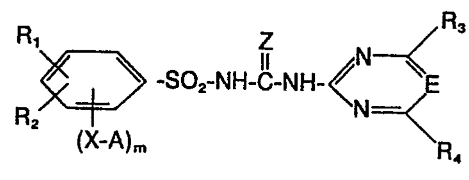 (I)

worin

A einen $C_3$–$C_6$-Alkinylrest,

E die Methingruppe oder Stickstoff,

X Sauerstoff, Schwefel, eine Sulfinyl- oder Sulfonylbrücke,

Z Sauerstoff oder Schwefel,

m die Zahl eins oder zwei,

$R_1$ Wasserstoff, Halogen, $C_1$–$C_5$-Alkyl, $C_2$–$C_5$-Alkenyl oder einen Rest –Y–$R_5$,

$R_2$ Wasserstoff, Halogen $C_1$–$C_5$-Alkyl, $C_2$–$C_5$-Alkenyl, $C_1$–$C_4$-Halogenalkyl, oder einen Rest –Y–$R_5$, –$COOR_6$, –$NO_2$ oder –CO–$NR_7$–$R_8$,

$R_3$ und $R_4$ unabhängig voneinander Wasserstoff, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, Halogen oder Alkoxyalkyl mit höchstens 4 Kohlenstoffatomen,

$R_5$ und $R_6$ je $C_1$–$C_5$-Alkyl, $C_2$–$C_5$-Alkenyl oder $C_2$–$C_6$-Alkinyl,

$R_7$ und $R_8$ unabhängig voneinander Wasserstoff, $C_1$–$C_5$-Alkyl, $C_2$–$C_5$-Alkenyl oder $C_2$–$C_6$-Alkinyl und

Y Sauerstoff, Schwefel, eine Sulfinyl- oder Sulfonylbrücke bedeuten, sowie die Salze dieser Verbindungen.

2. Verbindungen gemäss Anspruch 1, dadurch gekennzeichnet, dass Z Sauerstoff bedeutet.

3. Verbindungen gemäss Anspruch 2, dadurch gekennzeichnet, dass nur ein Rest –X–A in der 2-Stellung zum Sulfonylrest vorhanden ist und die Reste $R_3$ und $R_4$ zusammen nicht mehr als 4 Kohlenstoffatome enthalten.

4. Verbindungen gemäss Anspruch 2, dadurch gekennzeichnet, dass zwei Reste –X–A in 2- und 5-Stellung zum Sulfonylrest vorhanden sind und die Reste $R_3$ und $R_4$ zusammen nicht mehr als 4 Kohlenstoffatome enthalten.

5. Verbindungen gemäss Anspruch 3, dadurch gekennzeichnet, dass $R_1$ Wasserstoff bedeutet, und der Rest $R_2$ in der 5- oder 6-Stellung zur Sulfonylgruppe steht.

6. Verbindungen gemäss Anspruch 5, dadurch gekennzeichnet, dass $R_2$ Wasserstoff und $R_3$ und $R_4$ jeweils $C_1$–$C_4$-Alkyl, $C_1$–$C_4$-Alkoxy, Methylthio, 2,2,2-Trifluoräthoxy, Difluormethoxy, 1,1,2,2-Tetrafluoräthoxy, Halogen oder Alkoxyalkyl bedeuten.

7. Verbindungen gemäss Anspruch 6, dadurch gekennzeichnet, dass X für Sauerstoff steht.

8. Verbindungen gemäss Anspruch 7, dadurch gekennzeichnet, dass A für Propargyl steht.

9. Verbindungen gemäss Anspruch 1, dadurch gekennzeichnet, dass Z Schwefel, X Sauerstoff oder Schwefel, $R_3$ und $R_4$ jeweils unabhängig voneinander $C_1$–$C_3$-Alkyl, $C_1$–$C_3$-Alkoxy, $C_1$–$C_3$-Alkylthio, Difluormethoxy oder 2,2,2-Trifluoräthoxy mit zusammen höchstens 4 Kohlenstoffatomen bedeuten und A für $-CH_2-C\equiv CH$, $-CH_2-CH\equiv CH_3$, $-CH_2-CH_2-C\equiv CH$ steht und der Rest –X–A die 2-Stellung besetzt und m die Zahl 1 bedeutet.

10. N-(2-Propargyloxyphenylsulfonyl)-N'-(4-methoxy-6-methyl-1,3,5-triazin-2-yl)-harnstoff

gemäss Anspruch 1.

11. N-(2-Propargyloxyphenylsulfonyl)-N'-(4-äthyl-6-methoxy-1,3,5-triazin-2-yl)-harnstoff

gemäss Anspruch 1.

12. N-(2-Propargyloxyphenylsulfonyl)-N'-(4,6-dimethoxy-1,3,5-triazin-2-yl)-harnstoff

gemäss Anspruch 1.

13. N-(2-Propargyloxyphenylsulfonyl)-N'-(4-äthox-6-methyl-1,3,5-triazin-2-yl)-harnstoff

gemäss Anspruch 1.

14. Verfahren zur Herstellung der Verbindungen der Formel I, Anspruch 1, dadurch gekennzeichnet, dass man ein Phenylsulfonamid der Formel II

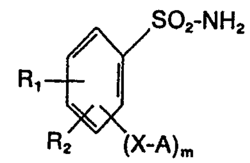 (II),

worin A, $R_1$, $R_2$, X und m die unter Formel I gegebene Bedeutung haben, in Gegenwart einer Base mit einem N-Pyrimidinyl- oder -Triazinylcarbamat der Formel

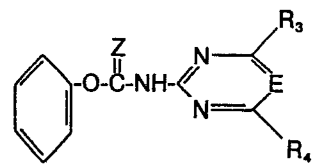 (III)

worin E, $R_3$, $R_4$ und Z die unter Formel I gegebene Bedeutung haben, umsetzt und gegebenenfalls in ihre Salze überführt.

15. Verfahren zur Herstellung der Verbindungen der Formel I, Anspruch 1, dadurch gekennzeichnet, dass man ein Phenylsulfonylisocyanat oder -isothiocyanat der Formel IV

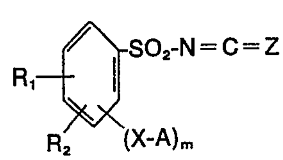 (IV),

worin A, $R_1$, $R_2$, m, X und Z die unter Formel I gegebene Bedeutung haben, gegebenenfalls in Gegenwart einer Base, mit einem Amin der Formel V

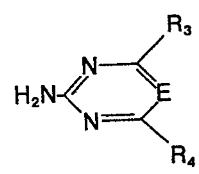 (V),

worin E, $R_3$ und $R_4$ die unter Formel I gegebene Bedeutung haben, umsetzt und gegebenenfalls in ihre Salze überführt.

16. Verfahren zur Herstellung der Verbindungen der Formel I, Anspruch 1, dadurch gekennzeichnet, dass man Sulfonamide der oben angegebenen Formel II gegebenenfalls in Gegenwart einer Base mit einem Isocyanat oder Isothiocyanat der Formel VI

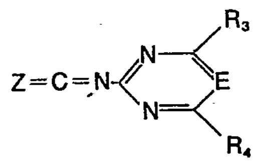 (VI),

worin E, $R_3$, $R_4$ und Z die unter Formel I gegebenen Bedeutungen haben, umsetzt und gegebenenfalls in ihre Salze überführt.

17. Verfahren zur Herstellung der Verbindungen der Formel I, Anspruch 1, dadurch gekennzeichnet, dass man ein N-Phenylsulfonylcarbamat der Formel VII

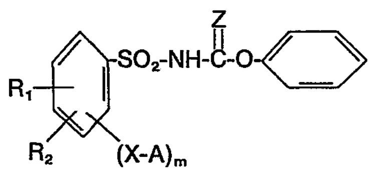 (VII)

worin A, $R_1$, $R_2$, m, X und Z die unter Formel I gegebene Bedeutung haben, mit einem Amin der oben angegebenen Formel V umsetzt und gegebenenfalls in ihre Salze überführt.

18. Verfahren zur Herstellung von Additionssalzen der Formel I gemäss einem der Ansprüche 14 bis 17, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

19. Phenylsulfonamide der Formel II

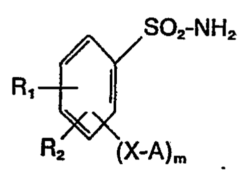 (II),

worin A, $R_1$, $R_2$, m und X die unter Formel I, Anspruch 1, gegebene Bedeutung haben.

20. N-Phenylsulfonylcarbamate der Formel VII

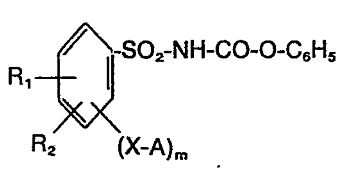 (VII)

worin A, $R_1$, $R_2$, m und X die unter Formel I, Anspruch 1, gegebene Bedeutung haben.

21. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

22. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

23. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

24. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwekke einer Ertragssteigerung.

25. Die Verwendung der N-Phenylsulfonyl-N'-triazinyl- oder -pyrimidinyl-harnstoffe der Formel I, oder sie enthaltender Mittel, gemäss Anspruch 22, zur selektiven pre- oder postemergenten Bekämpfung von Unkräutern in Nutzpflanzenkulturen.

26. Die Verwendung der N-Phenylsulfonyl-N'-triazinyl- oder -pyrimidinyl-harnstoffe der Formel I, oder sie enthaltender Mittel, gemäss Anspruch 23, zur Unterdrückung des Pflanzenwachstums über das 2-Blattstadium hinaus, dadurch gekennzeichnet, dass die Wirkstoffe preemergent angewendet werden.

27. Die Verwendung der Verbindungen der Formel I oder sie enthaltender Mittel gemäss Anspruch 25 im Getreide.

28. Die Verwendung der Verbindungen der Formel I oder sie enthaltender Mittel gemäss Anspruch 24 in Soja.

**Patentansprüche für den Vertragsstaat AT**

1. Ein herbizides und den Pflanzenwuchs hemmendes Mittel, dadurch gekennzeichnet, dass es neben Träger- und/oder anderen Zuschlagstoffen als Wirkstoff mindestens einen N-Phenylsulfonyl-N'-pyrimidinyl- und -triazinyl-harnstoff der allgemeinen Formel I

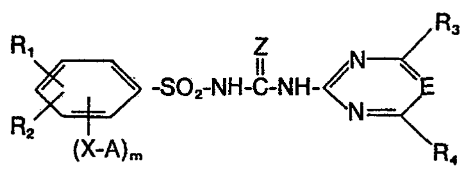 (I)

oder ein Salz dieser Verbindungen enthält, worin
A einen $C_3$–$C_6$-Alkinylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$–$C_5$-Alkyl, $C_2$–$C_5$-Alkenyl oder einen Rest $-Y-R_5$,
$R_2$ Wasserstoff, Halogen, $C_1$–$C_5$-Alkyl, $C_2$–$C_5$-Alkenyl, $C_1$–$C_4$-Halogenalkyl, oder einen Rest $-Y-R_5$, $-COOR_6$, $-NO_2$ oder $-CO-NR_7-R_8$,
$R_3$ und $R_4$ unabhängig voneinander Wasserstoff, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, Halogen oder Alkoxyalkyl mit höchstens 4 Kohlenstoffatomen,
$R_5$ und $R_6$ je $C_1$–$C_5$-Alkyl, $C_2$–$C_5$-Alkenyl oder $C_2$–$C_6$-Alkinyl,
$R_7$ und $R_8$ unabhängig voneinander Wasserstoff, $C_1$–$C_5$-Alkyl, $C_2$–$C_5$-Alkenyl oder $C_2$–$C_6$-Alkinyl und
Y Sauerstoff, Schwefel, eine Sulfinyl- oder Sulfonylbrücke bedeuten.

2. Mittel gemäss Anspruch 1, dadurch gekennzeichnet, dass Z Sauerstoff bedeutet.

3. Mittel gemäss Anspruch 2, dadurch gekennzeichnet, dass nur ein Rest –X–A in der 2-Stellung zum Sulfonylrest vorhanden ist und die Reste $R_3$ und $R_4$ zusammen nicht mehr als 4 Kohlenstoffatome enthalten.

4. Mittel gemäss Anspruch 2, dadurch gekennzeichnet, dass zwei Reste –X–A in 2- und 5-Stellung zum Sulfonylrest vorhanden sind und die Reste $R_3$ und $R_4$ zusammen nicht mehr als 4 Kohlenstoffatome enthalten.

5. Mittel gemäss Anspruch 3, dadurch gekennzeichnet, dass $R_1$ Wasserstoff bedeutet, und der Rest $R_2$ in der 5- oder 6-Stellung zur Sulfonylgruppe steht.

6. Mittel gemäss Anspruch 5, dadurch gekennzeichnet, dass $R_2$ Wasserstoff und $R_3$ und $R_4$ jeweils $C_1$–$C_4$-Alkyl, $C_1$–$C_4$-Alkoxy, Methylthio, 2,2,2-Trifluoräthoxy, Difluormethoxy, 1,1,2,2-Tetrafluoräthoxy, Halogen oder Alkoxyalkyl bedeuten.

7. Mittel gemäss Anspruch 6, dadurch gekennzeichnet, dass X für Sauerstoff steht.

8. Mittel gemäss Anspruch 7, dadurch gekennzeichnet, dass A für Propargyl steht.

9. Mittel gemäss Anspruch 1, dadurch gekennzeichnet, dass Z Schwefel, X Sauerstoff oder Schwefel, $R_3$ und $R_4$ jeweils unabhängig voneinander $C_1$–$C_3$-Alkyl, $C_1$–$C_3$-Alkoxy, $C_1$–$C_3$-Alkylthio, Difluormethoxy oder 2,2,2-Trifluoräthoxy mit zusammen höchstens 4 Kohlenstoffatomen bedeuten und A für $-CH_2-C\equiv CH$, $-CH_2-C\equiv C-CH_3$, $-CH_2-CH_2-C\equiv CH$ steht und der Rest –X–A die Stellung besetzt und m die Zahl 1 bedeutet.

10. Mittel gemäss Anspruch 1, dadurch gekennzeichnet, dass es als Wirkstoff
N-(2-Propargyloxyphenylsulfonyl)-N′-(4-methoxy-6-methyl-1,3,5-triazin-2-yl)-harnstoff
enthält.

11. Mittel gemäss Anspruch 1, dadurch gekennzeichnet, dass es als Wirkstoff
N-(2-Propargyloxyphenylsulfonyl)-N′-(4-äthyl-6-methoxy-1,3,5-triazin-2-yl)-harnstoff
enthält.

12. Mittel gemäss Anspruch 1, dadurch gekennzeichnet, dass es als Wirkstoff
N-(2-Propargyloxyphenylsulfonyl)-N′-(4,6-dimethoxy-1,3,5-triazin-2-yl)-harnstoff
enthält.

13. Mittel gemäss Anspruch 1, dadurch gekennzeichnet, dass es als Wirkstoff
N-(2-Propargyloxyphenylsulfonyl)-N′-(4-äthoxy-6-methyl-1,3,5-triazin-2-yl)-harnstoff
enthält.

14. Verfahren zur Herstellung der Wirkstoffe der Formel I, dadurch gekennzeichnet, dass man

a) ein Phenylsulfonamid der Formel II

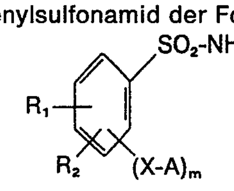
(II),

worin E, $R_3$, $R_4$ und Z die unter Formel I gegebene Bedeutung haben, in Gegenwart einer Base mit einem N-Pyrimidinyl- oder -Triazinylcarbamat der Formel

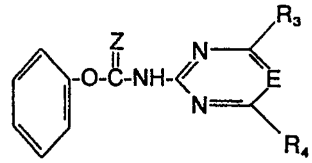
(III)

worin E, $R_3$, $R_4$ und Z die unter Formel I gegebene Bedeutung haben, umsetzt oder

b) ein Phenylsulfonylisocyanat oder -isothiocyanat der Formel IV

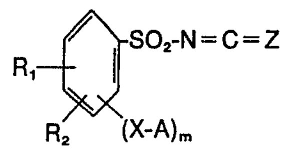
(IV),

worin A, $R_1$, $R_2$, m, X und Z die unter Formel I gegebene Bedeutung haben, gegebenenfalls in Gegenwart einer Base, mit einem Amin der Formel

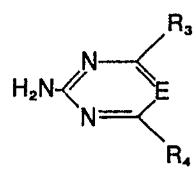
(V),

worin E, $R_3$ und $R_4$ die unter Formel I gegebene Bedeutung haben, umsetzt oder

c) Sulfonamide der oben angegebenen Formel II gegebenenfalls in Gegenwart einer Base mit einem Isocyanat oder Isothiocyanat der Formel VI

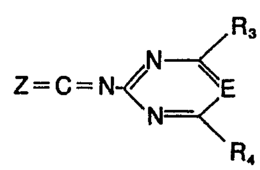
(VI),

worin E, $R_3$, $R_4$ und Z die unter Formel I gegebene Bedeutung haben, umsetzt oder

d) ein N-Phenylsulfonylcarbamat der Formel

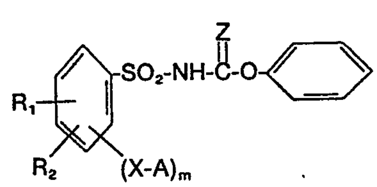
(VII)

worin A, $R_1$, $R_2$, m, X und Z die unter Formel I gegebene Bedeutung haben, mit einem Amin der oben angegebenen Formel V umsetzt und gegebenenfalls in ihre Salze überführt.

15. Verfahren zur Herstellung von Additionssalzen der Formel I gemäss Anspruch 14, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

16. Die Verwendung der N-Phenylsulfonyl-N′-triazinyl- oder -pyrimidinyl-harnstoffe der Formel I oder sie enthaltender Mittel zur Bekämpfung unerwünschten Pflanzenwachstums.

17. Die Verwendung der N-Phenylsulfonyl-N'-triazinyl- oder -pyrimidinyl-harnstoffe der Formel I oder sie enthaltender Mittel zur Hemmung des Pflanzenwachstums.

18. Die Verwendung der N-Phenylsulfonyl-N'-triazinyl- oder -pyrimidinyl-harnstoffe der Formel I oder sie enthaltender Mittel zur Wuchsregulierung von Kulturpflanzen zum Zwecke einer Ertragssteigerung.

19. Die Verwendung der N-Phenylsulfonyl-N'-triazinyl- oder -pyrimidinyl-harnstoffe der Formel I oder sie enthaltender Mittel, gemäss Anspruch 16, zur selektiven pre- oder postemergenten Bekämpfung von Unkräutern in Nutzpflanzenkulturen.

20. Die Verwendung der N-Phenylsulfonyl-N'-triazinyl- oder -pyrimidinyl-harnstoffe der Formel I oder sie enthaltender Mittel, gemäss Anspruch 17, zur Unterdrückung des Pflanzenwachstums über das 2-Blattstadium hinaus, dadurch gekennzeichnet, dass die Wirkstoffe preemergent angewendet werden.

21. Die Verwendung der Verbindungen der Formel I oder sie enthaltender Mittel gemäss Anspruch 19 im Getreide.

22. Die Verwendung der Verbindungen der Formel I oder sie enthaltender Mittel gemäss Anspruch 18 in Soja.

**Claims for the Contracting States: BE, CH, DE, FR, GB, IT, LI, NL**

1. An N-phenylsulfonyl-N'-pyrimidinyl- or -triazinylurea of the general formula I

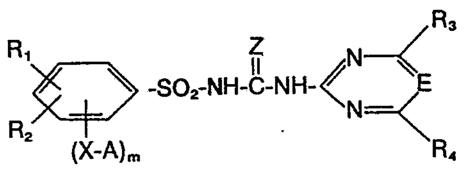

(I)

wherein

A is a $C_3$–$C_6$alkynyl group,

E is the methine group of nitrogen,

X is oxygen, sulfur or a sulfinyl or sulfonyl bridge,

Z is oxygen or sulfur,

m is one or two,

$R_1$ is hydrogen, halogen, $C_1$–$C_5$alkyl or $C_2$–$C_5$alkenyl or a group $-Y-R_5$,

$R_2$ is hydrogen, halogen, $C_1$–$C_5$alkyl, $C_2$–$C_5$alkenyl or $C_1$–$C_4$haloalkyl, or a group $-Y-R_5$, $-COOR_6$, $-NO_2$ or $-CO-NR_7-R_8$,

$R_3$ and $R_4$ are each independently of the other hydrogen, $C_1$–$C_4$alkyl, $C_1$–$C_4$alkoxy, $C_1$–$C_4$alkylthio, $C_1$–$C_4$haloalkyl, $C_1$–$C_4$haloalkoxy, halogen or alkoxyalkyl having at most 4 carbon atoms,

$R_5$ and $R_6$ are each $C_1$–$C_5$alkyl, $C_2$–$C_5$alkenyl or $C_2$–$C_6$alkynyl,

$R_7$ and $R_8$ are each independently of the other hydrogen, $C_1$–$C_5$alkyl, $C_2$–$C_5$alkenyl or $C_2$–$C_6$alkynyl, and

Y is oxygen, sulfur or a sulfinyl or sulfonyl bridge; or a salt thereof.

2. A compound according to claim 1, wherein Z is oxygen.

3. A compound according to claim 2, wherein only one –X–A group is in the 2-position with respect to the sulfonyl group, and $R_3$ and $R_4$ together contain not more than 4 carbon atoms.

4. A compound according to claim 2, wherein one –X–A group is in each of the 2- and 5-positions with respect to the sulfonyl group, and $R_3$ and $R_4$ together contain not more than 4 carbon atoms.

5. A compound according to claim 3, wherein $R_1$ is hydrogen, and $R_2$ is in the 5- or 6-position with respect to the sulfonyl group.

6. A compound according to claim 5, wherein $R_2$ is hydrogen, and $R_3$ and $R_4$ are each $C_1$–$C_4$alkyl, $C_1$–$C_4$alkoxy,methylthio, 2,2,2-trifluoroethoxy, difluoromethoxy, 1,1,2,2-tetrafluoroethoxy, halogen or alkoxyalkyl.

7. A compound according to claim 6, wherein X is oxygen.

8. A compound according to claim 7, wherein A is propargyl.

9. A compound according to claim 1, wherein Z is sulfur, X is oxygen or sulfur, $R_3$ and $R_4$ are each independently of the other $C_1$–$C_3$alkyl, $C_1$–$C_3$alkoxy, $C_1$–$C_3$alkylthio, difluoromethoxy or 2,2,2-trifluoroethoxy with together at most 4 carbon atoms, and A is $-CH_2-C\equiv CH$, $-CH_2-CH\equiv CH_3$, $-CH_2-CH_2-C\equiv CH$, and the –X–A group occupies the 2-position, and m is 1.

10. N-(2-Propargyloxyphenylsulfonyl)-N'-(4-methoxy-6-methyl-1,3,5-triazin-2-yl)urea according to claim 1.

11. N-(2-Propargyloxyphenylsulfonyl)-N'-(4-ethyl-6-methoxy-1,3,5-triazin-2-yl)urea according to claim 1.

12. N-(2-Propargyloxyphenylsulfonyl)-N'-(4,6-dimethoxy-1,3,5-triazin-2-yl)urea according to claim 1.

13. N-(2-Propargyloxyphenylsulfonyl)-N'-(4-ethoxy-6-methyl-1,3,5-triazin-2-yl)urea according to claim 1.

14. A process for the preparation of a compound of formula I according to claim 1, which process comprises reacting a phenylsulfonamide of formula II

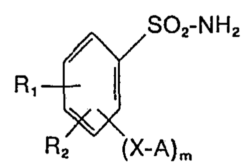

(II)

wherein A, $R_1$, $R_2$, X and m are as defined for formula I, in the presence of a base, with an N-pyrimidinyl- or -triazinylcarbamate of formula III

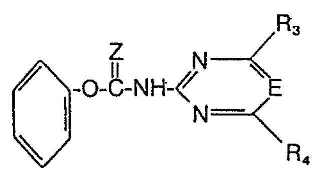

(III)

wherein E, $R_3$, $R_4$ and Z are as defined for formula I, and, of desired, converting the compound obtained into a salt thereof.

15. A process for preparation of a compound of formula I according to claim 1, which process comprises reacting a phenylsulfonylisocyanate or -isothiocyanate of formula IV

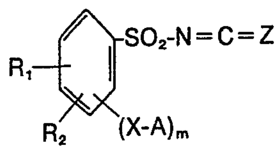

(IV)

wherein A, $R_1$, $R_2$, m, X and Z are as defined for formula I, in the absence or presence of a base, with an amine of formula V

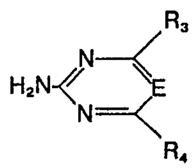

(V)

wherein E, $R_3$ and $R_4$ are as defined for formula I, and, if desired, converting the compound obtained into a salt thereof.

16. A process for the preparation of a compound of formula I according to claim 1, which process comprises reacting a sufonamide of formula II above, in the absence or presence of a base, with an isocyanate or isothiocyanate of formula VI

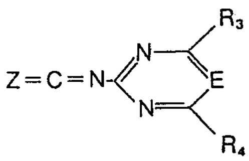

(VI)

wherein E, $R_3$, $R_4$ and Z are as defined for formula I, and, of desired, converting the compound obtained into a salt thereof.

17. A process for the preparation of a compound of formula I according to claim 1, which process comprises reacting an N-phenylsulfonylcarbamate of formula VII

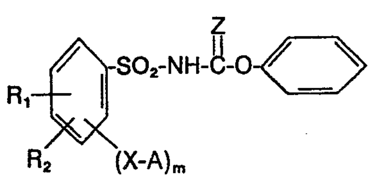

(VII)

wherein A, $R_1$, $R_2$, m, X and Z are as defined for formula I, with an amine of formula V above and, if desired, converting the compound obtained into a salt thereof.

18. A process for the preparation of an addition salt of formula I according to any one of claims 14 to 17, which process comprises reacting a sulfonylurea of formula I with an amine, an alkali metal hydroxide or alkaline earth metal hydroxide or a quaternary ammonium base.

19. A phenylsulfonamide of formula II

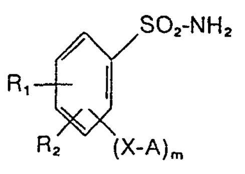

(II)

wherein A, $R_1$, $R_2$, m and X are as defined for formula I according to claim 1.

20. An N-phenylsulfonylcarbamate of formula VII

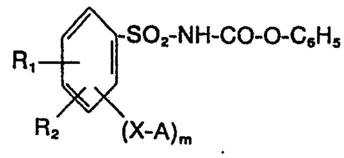

(VII)

wherein A, $R_1$, $R_2$, m and X are as defined for formula I according to claim 1.

21. A herbicidal and plant growth inhibiting composition which contains, as active ingredient, at least one N-phenylsulfonyl-N′-triazinyl- or -pyrimidinylurea of formula I according to claim 1, together with carriers and/or other adjuvants.

22. A method of combating undesirable plant growth, which method comprises applying thereto or to the locus thereof an effective amount of an N-phenylsulfonyl-N′-triazinyl- or -pyrimidinylurea of formula I according to claim 1, or of a composition containing such a compound.

23. A method of inhibiting plant growth, which method comprises applying thereto or to the locus thereof an effective amount of an N-phenylsulfonyl-N′-triazinyl- or -pyrimidinylurea of formula I, according to claim 1, or of a composition containing such a compound.

24. A method of regulating the growth of cultivated plants for the purpose of increasing the yield, which method comprises applying thereto or to the locus thereof an effective amount of an N-phenylsulfonyl-N′-triazinyl- or -pyrimidinylurea of formula I according to claim 1, or of a composition containing such a compound.

25. A method according to claim 22 of selectively combating weeds pre- or postemergence in crops of useful plants, which method comprises applying thereto or to the locus thereof an effective amount of an N-phenylsulfonyl-N′-triazinyl- or -pyrimidinylurea of formula I, or of a composition containing such a compound.

26. A method according to claim 23 of suppressing plant growth beyond the two-leaf stage, which method comprises applying preemergence an effective amount of an N-phenylsulfonyl-N′-triazinyl- or -pyrimidinylurea of formula I, or of a composition containing such a compound.

27. A method according to claim 25, which method comprises the use of a compound of formula I, or of a composition containing such a compound, in cereal crops.

28. A method according to claim 24, which method comprises the use of a compound of formula I, or of a composition containing such a compound, in soybean crops.

**Claims for the Contracting State: AT**

1. A herbicidal and plant growth inhibiting composition which contains, as active ingredient, at least one N-phenylsulfonyl-N′-pyrimidinyl- or -triazinylurea of the general formula I

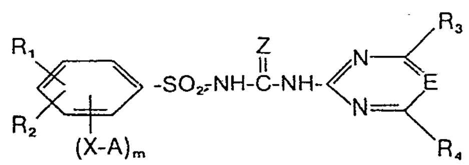 (I)

wherein

A is a $C_3$–$C_6$alkynyl group,

E is the methine group or nitrogen,

X is oxygen, sulfur or a sulfinyl or sulfonyl bridge,

Z is oxygen or sulfur,

n is one or two,

$R_1$ is hydrogen, halogen, $C_1$–$C_5$alkyl or $C_2$–$C_5$alkenyl or a group $-Y-R_5$,

$R_2$ is hydrogen, halogen, $C_1$–$C_5$alkyl, $C_2$–$C_5$alkenyl or $C_1$–$C_4$haloalkyl, or a group $-Y-R_5$, $-COOR_6$, $-NO_2$ or $-CO-NR_7-R_8$,

$R_3$ and $R_4$ each independently of the other hydrogen, $C_1$–$C_4$alkyl, $C_1$–$C_4$alkoxy, $C_1$–$C_4$alkylthio, $C_1$–$C_4$haloalkyl, $C_1$–$C_4$haloalkoxy, halogen or alkoxyalkyl having at most 4 carbon atoms,

$R_5$ and $R_6$ are each $C_1$–$C_5$alkenyl or $C_2$–$C_6$alkynyl,

$R_7$ and $R_8$ are each independently of the other hydrogen, $C_1$–$C_5$alkyl, $C_2$–$C_5$alkenyl or $C_2$–$C_6$alkynyl, and

X is oxygen, sulfur or a sulfinyl or sulfonyl bridge; or a salt thereof, together with carriers and/or other adjuvants.

2. A composition according to claim 1, wherein Z is oxygen.

3. A composition according to claim 2, wherein only one –X–A group is in the 2-position with respect to the sulfonyl group, and $R_3$ and $R_4$ together contain not more than 4 carbon atoms.

4. A composition according to claim 2, wherein one –X–A group is in each of the 2- and 5-positions with respect to the sulfonyl group, and $R_3$ and $R_4$ together contain not more than 4 carbon atoms.

5. A composition according to claim 3, wherein $R_1$ is hydrogen, and $R_2$ is in the 5- or 6-position with respect to the sulfonyl group.

6. A composition according to claim 5, wherein $R_2$ is hydrogen, and $R_3$ and $R_4$ are each $C_1$–$C_4$alkyl, $C_1$–$C_4$alkoxy, methylthio, 2,2,2-trifluoroethoxy, difluoromethoxy, 1,1,2,2-tetrafluoroethoxy, halogen or alkoxyalkyl.

7. A composition according to claim 6, wherein X is oxygen.

8. A composition according to claim 7, wherein A is propargyl.

9. A composition according to claim 1, wherein Z is sulfur, X is oxygen or sulfur, $R_3$ and $R_4$ are each independently of the other $C_1$–$C_3$alkyl, $C_1$–$C_3$alkoxy, $C_1$–$C_3$alkylthio, difluoromethoxy or 2,2,2-trifluoroethoxy with together at most 4 carbon atoms, and A ist $-CH_2-C\equiv CH$, $-CH_2-C\equiv CH_3$, $-CH_2-CH_2-C\equiv CH$, and the –X–A group occupies the 2-position, and m is 1.

10. A composition according to claim 1, which contains, as active ingredient, N-(2-propargyloxyphenylsulfonyl)-N′-(4-methoxy-6-methyl-1,3,5-triazin-2-yl)urea.

11. A composition according to claim 1, which contains, as active ingredient, N-(2-propargyloxyphenylsulfonyl)-N′-(4-ethyl-6-methoxy-1,3,5-triazin-2-yl)urea.

12. A composition according to claim 1, which contains, as active ingredient, N-(2-propargyloxyphenylsulfonyl)-N′-(4,6-dimethoxy-1,3,5-triazin-2-yl)urea.

13. A composition according to claim 1, which contains, as active ingredient, N-(2-propargyloxyphenylsulfonyl)-N′-(4-ethoxy-6-methyl-1,3,5-triazin-2-yl)urea.

14. A process for the preparation of a compound of formula I, which process comprises

a) reacting a phenylsulfonamide of formula II

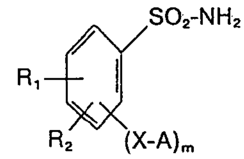 (II)

wherein A, $R_1$, $R_2$, X and m are as defined for formula I, in the presence of a base, with an N-pyrimidinyl- or -triazinylcarbamate of formula III

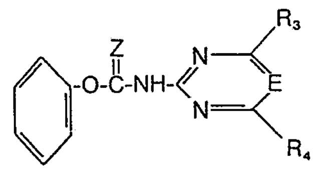 (III)

wherein E, $R_3$, $R_4$ and Z are defined for formula I,

b) reacting a phenylsulfonylisocyanate or -isothiocyanate of formula IV

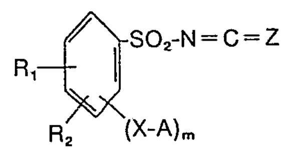 (IV)

wherein A, $R_1$, $R_2$, m, X and Z are as defined for formula I, in the absence or presence of a base, with an amine of formula V

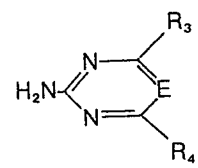 (V),

wherein E, $R_3$ and $R_4$ are as defined for formula I,

c) reacting a sulfonamide of formula II above, in the absence or presence of a base, with an isocyanate or isothiocyanate of formula VI

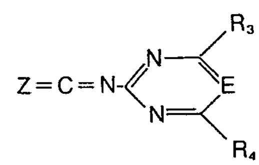 (VI)

wherein E, $R_3$, $R_4$ and Z are as defined for formula I, or

d) reacting an N-phenylsulfonylcarbamate of formula VII

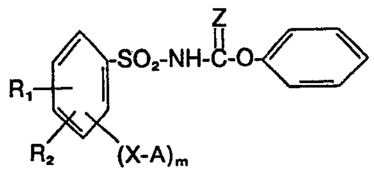

(VII)

wherein A, $R_1$, $R_2$, m, X and Z are as defined for formula I, with an amine of formula V above and, if desired, converting the compound obtained into a salt thereof.

15. A process for the preparation of an addition salt of formula I according to claim 14, which process comprises reacting a sulfonylurea of formula I with an amine, an alkali metal hydroxide or alkaline earth metal hydroxide or a quaternary ammonium base.

16. A method of combating undesirable plant growth, which method comprises applying thereto or to the locus thereof an effective amount of an N-phenylsulfonyl-N′-triazinyl- or -pyrimidinylurea of formula I, or of a composition containing such a compound.

17. A method of inhibiting plant growth, which method comprises applying thereto or to the locus thereof an effective amount of an N-phenylsulfonyl-N′-triazinyl- or -pyrimidinylurea of formula I, or of a composition containing such a compound.

18. A method of regulating the growth of cultivated plants for the purpose of increasing the yield, which method comprises applying thereto or to the locus thereof an effective amount of an N-phenylsulfonyl-N′-triazinyl- or -pyrimidinylurea of formula I, or of a composition containing such a compound.

19. A method according to claim 16 of selectively combating weeds pre- or postemergence in crops of useful plants, which method comprises applying thereto or to the locus thereof an effective amount of an N-phenylsulfonyl-N′-triazinyl- or -pyrimidinylurea of formula I, or of a composition containing such a compound.

20. A method according to claim 17 of suppressing plant growth beyond the two-leaf stage, which method comprises applying preemergence an effective amount of an N-phenylsulfonyl-N′-triazinyl- or -pyrimidinylurea of formula I, or of a composition containing such a compound.

21. A method according to claim 19, which method comprises the use of a compound of formula I, or of a composition containing such a compound, in cereal crops.

22. A method according to claim 18, which method comprises the use of a compound of formula I, or of a composition containing such a compound, in soybean crops.

**Revendications pour les Etats contractants: BE, CH, DE, FR, GB, IT, LI, NL**

1. N-Phénylsulfonyl-N′-pyrimidinyl- et -triazinylurées de formule générale I

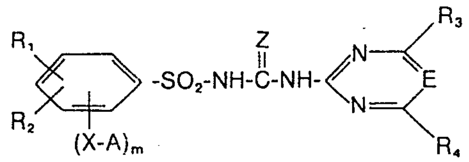

(I)

dans laquelle

A représente un groupe alcynyle en C3–C6,

E représente le groupe méthine ou l'azote,

X représente l'oxygène, le soufre, un pont sulfinyle ou sulfonyle,

Z représente l'oxygène ou le soufre,

m est égal à 1 ou 2,

$R_1$ représente l'hydrogène, un halogène, un groupe alkyle en C1–C5, alcényle en C2–C5 ou un groupe $-Y-R_5$,

$R_2$ représente l'hydrogène, un halogène, un groupe alkyle en C1–C5, alcényle en C2–C5, halogénoalkyle en C1–C4 ou un groupe $-Y-R_5$, $-COOR_6$, $-NO_2$ ou $-CO-NR_7-R_8$,

$R_3$ et $R_4$ représentent chacun, indépendamment l'un de l'autre, l'hydrogène, un groupe alkyle en C1–C4, alcoxy en C1–C4, alkylthio en C1–C4, halogéno-alkyle en C1–C4, halogénoalcoxy en C1–C4, une halogène ou un groupe alcoxyalkyle contenant au maximum 4 atomes de carbone,

$R_5$ et $R_6$ représentent chacun un groupe alkyle en C1–C5, alcényle en C2–C5 ou alcynyle en C2–C6,

$R_7$ et $R_8$ reprèsentent chacun, indépendamment l'un de l'autre, l'hydrogène, un groupe alkyle en C1–C5, alcényle en C2–C5 ou alcynyle en C2–C6 et

Y représente l'oxygène, le soufre, un pont sulfinyle ou sulfonyle,

et les sels de ces composés.

2. Composés selon la revendication 1, caractérisés en ce que Z représente l'oxygène.

3. Composés selon la revendication 2, caractérisés en ce qu'il n'y a qu'un seul groupe –X–A en position 2 par rapport au groupe sulfonyle et en ce que les groupes $R_3$ et $R_4$, ensemble, en contiennent pas plus de 4 atomes de carbone.

4. Composés selon la revendication 2, caractérisés en ce qu'il y a deux groupes –X–A en position 2 et en position 5 par rapport au groupe sulfonyle et en ce que les groupes $R_3$ et $R_4$, ensemble, ne contiennent pas plus de 4 atomes de carbone.

5. Composés selon la revendication 3, caractérisés en ce que $R_1$ représente l'hydrogène et en ce que le groupe $R_2$ est en position 5 ou 6 par rapport au groupe sulfonyle.

6. Composés selon la revendication 5, caractérisés en ce que $R_2$ représente l'hydrogène et $R_3$ et $R_4$ représentent chacun un groupe alkyle en C1–C4, alcoxy en C1–C4, méthylthio, 2,2,2-trifluoréthoxy, difluorométhoxy, 1,1,2,2-tétrafluoréthoxy, un halogène ou un groupe alcoxyalkyle.

7. Composés selon la revendication 6, caractérisés en ce que X représente l'oxygène.

8. Composés selon la revendication 7, caractérisés en ce que A représente un groupe propargyle.

9. Composés selon la revendication 1, caractérisés en ce que Z représente le soufre, X représente l'oxygène ou le soufre, $R_3$ et $R_4$ représentent cha-

cun, indépendamment l'un de l'autre, un groupe alkyle en C1–C3, alcoxy en C1–C3, alkylthio en C1–C3, difluorométhoxy ou 2,2,2-trifluoréthoxy contenant ensemble, au maximum, 4 atomes de carbone, et A représente $-CH_2-C\equiv CH$, $-CH_2-C\equiv C-CH_3$, $-CH_2-CH_2-C\equiv CH$, le groupe –X–A occupe la position 2 et m est égal à 1.

10. La N-(2-propargyloxyphénylsulfonyl)-N'-(4-méthoxy-6-méthyl-1,3,5-triazine-2-yl)-urée selon la revendication 1.

11. La N-(2-propargyloxyphénylsulfonyl)-N'-(4-éthyl-6-méthoxy-1,3,5-triazine-2-yl)-urée selon la revendication 1.

12. La N-(2-propargyloxyphénylsulfonyl)-N'-(4,6-diméthoxy-1,3,5-triazine-2-yl)-urée selon la revendication 1.

13. La N-(2-propargyloxyphénylsulfonyl)-N'-(4-éthoxy-6-méthyl-1,3,5-triazine-2-yl)-urée selon la revendication 1.

14. Procédé de préparation des composés de formule I, revendication 1, caractérisé en ce que l'on fait réagir un phénylsulfonamide de formule II

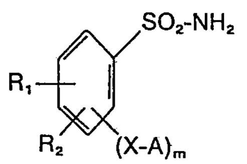

(II)

dans laquelle A, $R_1$, $R_2$, X et m ont les significations indiquées en référence à la formule I, en présence d'une base, avec un N-pyrimidinyl- ou -triazinyl-carbamate de formule

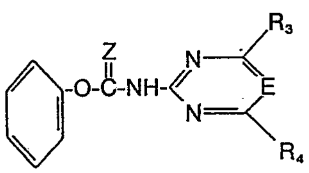

(III)

dans laquelle E, $R_3$, $R_4$ et Z ont les significations indiquées en référence à la formule I, et le cas échéant on convertit les composés obtenus en leurs sels.

15. Procédé de préparation des composés de formule I, revendication 1, caractérisé en ce que l'on fait réagir un phénylsulfonylisocyanate ou -isothiocyanate de formule IV

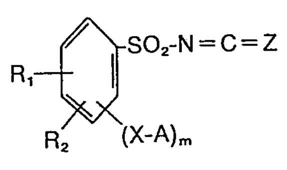

(IV)

dans laquelle A, $R_1$, $R_2$, m, X et Z ont les significations indiquées en référence à la formule I, éventuellement en présence d'une base, avec une amine de formule V

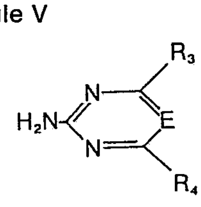

(V)

dans laquelle E, $R_3$ et $R_4$ ont les significations indiquées en référence à la formule I, et le cas échéant on convertit les composés obtenus en leurs sels.

16. Procédé de préparation des composés de formule I, revendication 1, caractérisé en ce que l'on fait réagir des sulfonamides de formule II ci-dessus, éventuellement en présence d'une base, avec un isocyanate ou isothiocyanate de formule VI

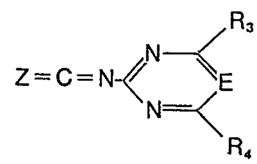

(VI)

dans laquelle E, $R_3$, $R_4$ et Z ont les significations indiquées en référence à la formule I, et le cas échéant on convertit les composés obtenus en leurs sels.

17. Procédé de préparation des composés de formule I, revendication 1, caractérisé en ce que l'on fait réagir un N-phénylsulfonylcarbamate de formule VII

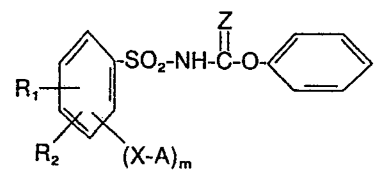

(VII)

dans laquelle A, $R_1$, $R_2$, m, X et Z ont les significations indiquées en référence à la formule I, avec une amine de formule V ci-dessus, et le cas échéant on convertit les composés obtenus en leurs sels.

18. Procédé de préparation de sels d'addition de formule I selon l'une des revendications 14 à 17, caractérisé en ce que l'on fait réagir une sulfonylurée de formule I avec une amine, un hydroxyde de métal alcalin ou alcalino-terreux ou une base d'ammonium quaternaire.

19. Phénylsulfonamides de formule II

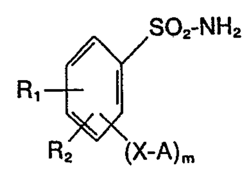

(II)

dans laquelle A, $R_1$, $R_2$, m et X ont les significations indiquées en référence à la formule I, revendication 1.

20. N-phénylsulfonylcarbamates de formule VII

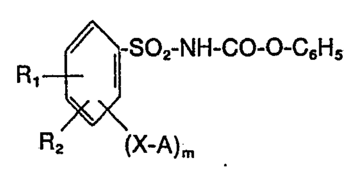

(VII)

dans laquelle A, $R_1$, $R_2$, m et X ont les significations indiquées en référence à la formule I, revendication 1.

21. Un produit herbicide et inhibiteur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, revendication 1.

22. L'utilisation des N-phénylsulfonyl-N'-triazinyl- ou -pyrimidinyl-urées de formule I, revendication 1, ou de produits en contenant, dans la lutte contre la croissance de végétaux indésirables.

23. L'utilisation des N-phénylsulfonyl-N'-triazinyl- ou -pyrimidinyl-urées de formule I, revendication 1, ou de produits en contenant, pour l'inhibition de la croissance des végétaux.

24. L'utilisation des N-phénylsulfonyl-N'-triazinyl- ou -pyrimidinyl-urées de formule I, revendication 1, ou de produits en contenant, pour la régulation de la croissance des végétaux cultivés en vue d'une augmentation de la récolte.

25. L'utilisation des N-phénylsulfonyl-N'-triazinyl- ou -pyrimidinyl-urées de formule I ou de produits en contenant, selon la revendication 22, pour la lutte sélective en pré-levée ou en post-levée contre les mauvaises herbes dans les cultures de végétaux utiles.

26. L'utilisation des N-phénylsulfonyl-N'-triazinyl- ou -pyrimidinyl-urées de formule I ou de produits en contenant, selon la revendication 23, pour l'inhibition de la croissance des végétaux au delà du stade deux feuilles, caractérisée en ce que l'on applique les substances actives en pré-levée.

27. L'utilisation des composés de formule I ou de produits en contenant, selon la revendication 25, dans les céréales.

28. L'utilisation des composés de formule I ou de produits en contenant, selon la revendication 24, dans le soya.

**Revendications pour l'Etat contractant: AT**

1. Un produit herbicide et inhibiteur de la croissance des végétaux, caractérisé en ce qu'il contient en tant que substance active, avec des véhicules et/ou d'autres additifs, au moins une N-phénylsulfonyl-N'-pyrimidinyl- et -triazinyl-urée de formule générale I

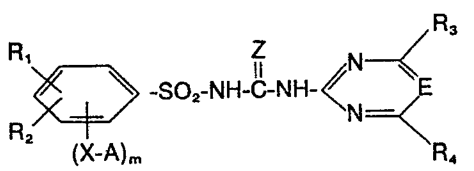

(I)

ou un sel d'un tel composé, dans lesquels

A représente un groupe alcynyle en C3–C6,

E représente le groupe méthine ou l'azote,

X représente l'oxygène, le soufre, un point sulfinyle ou sulfonyle,

Z représente l'oxygène ou le soufre,

m est égal à 1 ou 2,

$R_1$ représente l'hydrogène, un halogène, un groupe alkyle en C1–C5, alcényle en C2–C5 ou un groupe $-Y-R_5$,

$R_2$ représente l'hydrogène, un halogène, un groupe alkyle en C1–C5, alcényle en C2–C5, halogénoalkyle en C1–C4 ou groupe $-Y-R_5$, $-COOR_6$, $-NO_2$ ou $-CO-NR_7-R_8$,

$R_3$ et $R_4$ représentent chacun, indépendamment l'un de l'autre, l'hydrogène, un groupe alkyle en C1–C4, alcoxy en C1–C4, alkylthio en C1–C4, halogénoalkyle en C1–C4, halogénoalcoxy en C1–C4, un halogène ou un groupe alcoxyalkyle contenant au maximum 4 atomes de carbone,

$R_5$ et $R_6$ représentent chacun un groupe alkyle en C1–C5, alcényle en C2–C5 ou alcynyle en C2–C6,

$R_7$ et $R_8$ représentent chacun, indépendamment l'un de l'autre, l'hydrogène, un groupe alkyle en C1–C5, alcényle en C2–C5 ou alcynyle en C2–C6 et

Y représente l'oxygène, le soufre, un pont sulfinyle ou sulfonyle.

2. Produit selon la revendication 1, caractérisé en ce que Z représente l'oxygène.

3. Produit selon la revendication 2, caractérisé en ce qu'il n'y a qu'un seul groupe –X–A dans la position 2 par rapport au groupe sulfonyle et en ce que les groupes $R_3$ et $R_4$, ensemble, ne contiennent pas plus de 4 atomes de carbone.

4. Produit selon la revendication 2, caractérisé en ce qu'il y a deux groupes –X–A en position 2 et en position 5 par rapport au groupe sulfonyle et en ce que les groupes $R_3$ et $R_4$, ensemble, ne contiennent pas plus de 4 atomes de carbone.

5. Produit selon la revendication 3, caractérisé en ce que $R_1$ représente l'hydrogène et en ce que le groupe $R_2$ est en position 5 ou 6 par rapport au groupe sulfonyle.

6. Produit selon la revendication 5, caractérisé en ce que $R_2$ représente l'hydrogène et $R_3$ et $R_4$ représentent chacun un groupe alkyle en C1–C4, alcoxy en C1–C4, méthylthio, 2,2,2-trifluoréthoxy, difluorométhoxy, 1,1,2,2-tétrafluoréthoxy, un halogène ou un groupe alcoxyalkyle.

7. Produit selon la revendication 6, caractérisée en ce que X représente l'oxygène.

8. Produit selon la revendication 7, caractérisé en ce que A représente un groupe propargyle.

9. Produit selon la revendication 1, caractérisé en ce que Z représente le soufre, X représente l'oxygène ou le soufre, $R_3$ et $R_4$ représentent chacun, indépendamment l'un de l'autre, un groupe alkyle en C1–C3, alcoxy en C1–C3, alkylthio en C1–C3, difluorométhoxy ou 2,2,2-trifluoréthoxy contenant ensemble au maximum 4 atomes de carbone et A représente $-CH_2-C\equiv CH$, $-CH_2-C\equiv C-CH_3$, $-CH_2-CH_2-C\equiv CH$ et le groupe –X–A occupe la position 2 et m est égal à 1.

10. Produit selon la revendication 1, caractérisé en ce qu'il contient en tant que substance active la
N-(2-propargyloxyphénylsulfonyl)-N'-(4-méthoxy-6-méthyl-1,3,5-triazine-2-yl)-urée.

11. Produit selon la revendication 1, caractérisé en ce qu'il contient en tant que substance active la
N-(2-propargyloxyphénylsulfonyl)-N'-(4-éthyl-6-méthoxy-1,3,5-triazine-2-yl)-urée.

12. Produit selon la revendication 1, caractérisé en ce qu'il contient en tant que substance active la N-(2-propargyloxyphénylsulfonyl)-N'-(4,6-diméthoxy-1,3,5-triazine-2-yl)-urée.

13. Produit selon la revendication 1, caractérisé en ce qu'il contient en tant que substance active la N-(2-propargyloxyphénylsulfonyl)-N'-(4-éthoxy-6-méthyl-1,3,5-triazine-2-yl)-urée.

14. Procédé de préparation des substances actives de formule I, caractérisé en ce que

a) on fait réagir un phénylsulfonamide de formule II

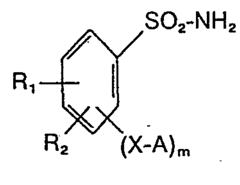
(II)

dans laquelle A, $R_1$, $R_2$, X et m ont les significations indiquées en référence à la formule I, en présence d'une base, avec un N-pyrimidinyl- ou -triazinyl-carbamate de formule

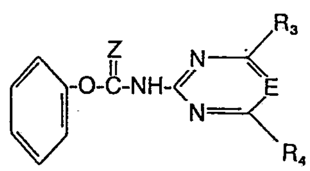
(III)

dans laquelle E, $R_3$, $R_4$ et Z ont les significations indiquées en référence à la formule I, ou bien

b) on fait réagir un phénylsulfonylisocyanate ou -isothiocyanate de formule IV

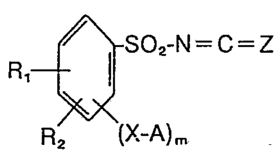
(IV)

dans laquelle A, $R_1$, $R_2$, m, X et Z ont les significations indiquées en référence à la formule I, éventuellement en présence d'une base, avec une amine de formule

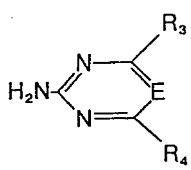
(V)

dans laquelle E, $R_3$ et $R_4$ ont les significations indiquées en référence à la formule I, ou bien

c) on fait réagir des sulfonamides de formule II ci-dessus, éventuellement en présence d'une base, avec un isocyanate ou isothiocyanate de formule VI

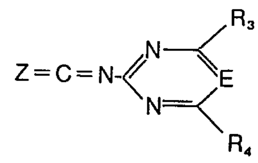
(VI)

dans laquelle E, $R_3$, $R_4$ et Z ont les significations indiquées en référence à la formule I, ou bien

d) on fait réagir un N-phénylsulfonylcarbamate de formule

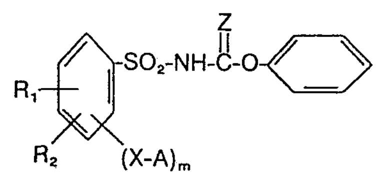
(VII)

dans laquelle A, $R_1$, $R_2$, m, X et Z ont les significations indiquées en référence à la formule I, avec une amine de formule V ci-dessus, et on convertit éventuellement les composés obtenus en leurs sels.

15. Procédé de préparation de sels d'addition de formule I selon la revendication 14, caractérisé en ce que l'on fait réagir une sulfonylurée de formule I avec une amine, un hydroxyde de métal alcalin ou alcalino-terreux ou une base d'ammonium quaternaire.

16. L'utilisation des N-phénylsulfonyl-N'-triazinyl- ou -pyrimidinyl-urées de formule I ou de produits en contenant dans la lutte contre les croissances de végétaux indésirables.

17. L'utilisation des N-phénylsulfonyl-N'-triazinyl- ou -pyrimidinyl-urées de formule I ou de produits en contenant pour l'inhibition de la croissance des végétaux.

18. L'utilisation des N-phénylsulfonyl-N'-triazinyl- ou -pyrimidinyl-urées de formule I ou de produits en contenant pour la régulation de la croissance des végétaux cultivés en vue d'une augmentation de la récolte.

19. L'utilisation des N-phénylsulfonyl-N'-triazinyl- ou -pyrimidinyl-urées de formule I ou de produits en contenant, selon la revendication 16, pour la lutte sélective en pré-levée ou en post-levée contre les mauvaises herbes dans les cultures de végétaux utiles.

20. L'utilisation des N-phénylsulfonyl-N'-triazinyl- ou -pyrimidinyl-urées de formule I ou de produits en contenant, selon la revendication 17, pour l'inhibition de la croissance des végétaux au-delà du stade deux feuilles, caractérisée en ce que l'on applique les substances actives en pré-levée.

21. L'utilisation des composés de formule I ou de produits en contenant selon la revendication 19 dans les céréales.

22. L'utilisation des composés de formule I ou de produits en contenant selon la revendication 18 dans le soya.